# EUROPEAN PATENT SPECIFICATION

(11) **EP 3 101 014 B1**
(45) Date of publication and mention of the grant of the patent: **13.03.2019**
(21) Application number: 15743007.5
(22) Date of filing: 28.01.2015
(51) Int. Cl.: C07D 307/79, C07D 307/80, C07D 307/81, C07C 43/23, A61K 31/343, A61K 31/085, A61K 31/09, A61P 29/00

(54) **NOVEL 2-PHENYLBENZOFURAN DERIVATIVE OR PHARMACEUTICALLY ACCEPTABLE SALT THEREOF, PRODUCTION METHOD FOR SAME AND PHARMACEUTICAL COMPOSITION FOR PREVENTING OR TREATING INFLAMMATORY DISEASE COMPRISING SAME AS ACTIVE INGREDIENT**
NEUARTIGES 2-PHENYLBENZOFURAN-DERIVAT ODER PHARMAZEUTISCH AKZEPTABLES SALZ DAVON, HERSTELLUNGSVERFAHREN DAFÜR UND PHARMAZEUTISCHE ZUSAMMENSETZUNG ZUR VORBEUGUNG ODER BEHANDLUNG VON ENTZÜNDLICHEN KRANKHEITEN DAMIT ALS WIRKSTOFF
NOUVEAU DÉRIVÉ 2-PHÉNYLBENZOFURANNE OU SEL PHARMACEUTIQUEMENT ACCEPTABLE ASSOCIÉ, SON PROCÉDÉ DE PRODUCTION ET COMPOSITION PHARMACEUTIQUE DE PRÉVENTION OU DE TRAITEMENT D'UNE MALADIE INFLAMMATOIRE COMPRENANT UN TEL COMPOSÉ UTILISÉ COMME PRINCIPE ACTIF

(30) Priority: 28.01.2014 KR 20140010224
(43) Date of publication of application: 07.12.2016
(73) Proprietor: Korea Research Institute of Bioscience and Biotechnology, Daejeon 305-806 (KR); Dongguk University Industry-Academic Cooperation Foundation, Seoul 100-715 (KR)
(72) Inventor: LEE, Kyeong, Seoul 100-715 (KR); OH, Sei-Ryang, Daejeon 305-806 (KR); AHN, Kyung Seop, Daejeon 305-806 (KR); KWON, Ok-Kyoung, Daejeon 305-806 (KR); KIM, Doo-Young, Daejeon 305-806 (KR); RYU, Hyung Won, Daejeon 305-806 (KR); KIM, Jung Hee, Daejeon 305-806 (KR); XU, Xuezhen, Goyang-si Gyeonggi-do 410-820 (KR)
(74) Representative: Abel & Imray
(86) International application number: PCT/KR2015/000922
(87) International publication number: WO 2015/115805

(56) References cited:
- EP-A2- 2 641 604
- WO-A1-2015/137775
- WO-A2-2006/023778
- JP-A- H11 158 067
- JUNG-WOON HWANG ET AL: "Facile Preparation of 2-Arylbenzo[b]furan Molecules and Their Anti-inflammatory Effects", BULLETIN OF THE KOREAN CHEMICAL SOCIETY, vol. 31, no. 4, 20 April 2010 (2010-04-20) , pages 965-970, XP055217742, ISSN: 0253-2964, DOI: 10.5012/bkcs.2010.31.04.965
- CAMILA S. BERTANHA ET AL: "Cyclooxygenase inhibitory properties of nor -neolignans from Styrax pohlii", NATURAL PRODUCT RESEARCH, vol. 26, no. 24, 29 March 2012 (2012-03-29), pages 2323-2329, XP055305503, GB ISSN: 1478-6419, DOI: 10.1080/14786419.2012.671320
- SCAMMELLS P J ET AL: "XH-14 analogs as adenosine antagonists", BIOORGANIC & MEDICINAL CHEMISTRY, PERGAMON, GB, vol. 6, no. 9, 1 January 1998 (1998-01-01) , pages 1517-1524, XP002986360, ISSN: 0968-0896, DOI: 10.1016/S0968-0896(98)00093-5
- ZHEN ; YANG ET AL: "Regioselective Introduction of Carbon-3 Substituents to 5-Alkyl-7-methoxy-2-phenylbenzo[ b ]furans: Synthesis of a Novel Adenosine Al Receptor Ligand and Its Derivatives'", JOURNAL OF ORGANIC CHEMISTRY, vol. 57, no. 26, 1 December 1992 (1992-12-01), pages 7248-7257, XP055305748,
- HEMPFLING R ET AL: "CHEMICAL CHARACTERIZATION OF THE ORGANIC MATTER IN FOREST SOILS BY CURIE POINT PYROLYSIS-GC/MS AND PYROLYSIS-FIELD IONIZATION MASS SPECTROMETRY", ORGANIC GEOCHEMISTRY, ELMSFORD, NY, US, vol. 15, no. 2, 1 January 1990 (1990-01-01), pages 131-145, XP002934711, DOI: 10.1016/0146-6380(90)90078-E
- KAO C-L ET AL: "A convenient synthesis of naturally occurring benzofuran ailanthoidol", TETRAHEDRON LETTERS, PERGAMON, GB, vol. 42, no. 6, 5 February 2001 (2001-02-05), pages 1111-1113, XP004316698, ISSN: 0040-4039, DOI: 10.1016/S0040-4039(00)02163-8
- DATABASE REGISTRY [Online] CHEMICAL ABSTRACTS SERVICE, COLUMBUS, OHIO, US; 17 November 2006 (2006-11-17), Database: ChemBank (The Broad Institute): XP002762303, Database accession no. 913485-10-6
- DEL RÍO J C ET AL: "Structural Characterization of Guaiacyl-rich Lignins in Flax (Linum usitatissimum) Fibers and Shives", JOURNAL OF AGRICULTURAL AND FOOD CHEMISTRY, AMERICAN CHEMICAL SOCIETY, US, vol. 59, no. 20, 9 September 2011 (2011-09-09), pages 11088-11099, XP002722561, ISSN: 0021-8561, DOI: 10.1021/JF201222R [retrieved on 2011-09-09]
- J.-W. HWANG ET AL.: 'Bull. Korean Chem. Soc.' FACILE PREPARATION OF 2-ARYLBENZO[B]FURAN MOLECULES AND THEIR ANTI-INFLAMMATORY EFFECTS vol. 31, no. 4, 2010, pages 965 - 970, XP055217742
- H.-Y. SUNG ET AL.: 'Chemico-Biological Interactions' NOVEL DANSHEN METHOAYBENZO[B] FURAN DERIVATIVE ANTAGONIZING ADIPOGENIC DIFFERENTIATION AND PRODUCTION OF INFLAMMATORY ADIPOKINES vol. 188, no. 3, 2010, pages 457 - 466, XP055217744
- C.-L. KAO ET AL.: 'A convenient synthesis of naturally occurring benzofuran ailanthoidol' TETRAHEDRON LETTERS vol. 42, no. 6, 2001, pages 1111 - 1113, XP004316698

## Description

### BACKGROUND OF THE INVENTION

### 1. Field of the Invention

The present invention relates to a novel 2-phenylbenzofuran derivative or a pharmaceutically acceptable salt thereof, a production method for the same, and a pharmaceutical composition for preventing or treating an inflammatory disease comprising the same as an active ingredient

### 2. Description of the Related Art

Inflammation is a kind of defense reaction in a living body against a foreign infectious agent including a physical •chemical stimulus, bacteria, fungi, virus, and various allergens. Inflammatory response is a part of innate immune response. Like in animals, the innate immune response in human starts with the macrophage's attack to a pathogen when the macrophage recognizes the pathogen as a non-self by catching a specific pattern on the cell surface of the pathogen. In the course of inflammatory response, blood plasma is accumulated in the area of inflammation to dilute the toxicity caused by bacteria, and also blood flow increases and such symptoms as erythema, pain, edema, and fever accompany.

Various biochemical phenomena are involved in the inflammatory response. In particular, it is known that nitric oxide synthase (NOS) and cyclooxygenase (COX) involved in the biosynthesis of various prostaglandins are important mediators of inflammatory response.

NOS has three isomers, which are calcium or camodulin dependent eNOS (endothelial NOS) and nNOS (neuronal NOS), and iNOS (inducible NOS) induced by bacterial endotoxin such as LPS (lipopolysaccharide) and various inflammatory cytokines such as IL-1β, TNF-α, IL-6, IL-8, and IL-12. They produce nitric oxide (NO) from L-arginine.

Nitric oxide (NO) generated by eNOS or nNOS is involved in various physiological responses including blood pressure regulation, neurotransmission, learning, and memory, indicating that NO plays an important role in maintaining homeostasis in a human body. In the meantime, NO generated by iNOS is involved in various inflammatory diseases such as arthritis, sepsis, graft rejection, autoimmune disease, and neuronal death (non-patent references 1 and 2) .

LPS, one of those components forming a cell wall of gram-negative bacteria, is able to activate macrophages, due to which it is often used for an inflammatory model. Once macrophages are activated by LPS, inflammation precursors such as cytokines, chemokines, and NO are secreted, which sometimes even show a cancerous activity. The over-expressions of those inflammation mediators such as cytokines, chemokines, and NO are observed in various diseases including sepsis, rheumatoid arthritis, autoimmune disease, and diabetes. So, a substance that can regulate the inflammatory response by controlling macrophages can be an important target for the treatment of inflammatory disease.

Therefore, a substance that can suppress the over-expressions of such inflammation mediators as described above can be developed as a therapeutic agent for the treatment of inflammatory disease.

Thus, the present inventors tried to develop a compound that could suppress nitric oxide (NO), IL-6, and TNF-alpha in macrophages. In the course of our study, the present inventors confirmed that a 2-phenylbenzofuran derivative with a specific structure was excellent in suppressing NO, IL-6, and TNF alpha, and thereby confirmed that the 2-phenylbenzofuran derivative can be effectively used as a composition for preventing and treating inflammatory disease, leading to the completion of this invention.

### [PRIOR ART REFERENCE]

### [NON-PATENT REFERENCE]

Moncade S. et al, Pharmacol. Rev., 1991, 43, 109;

Perreault M. and Marette A. Nature Medicine, 2001, 7, 1138;

### SUMMARY OF THE INVENTION

It is an object of the present invention to provide a novel 2-phenylbenzofuran derivative or a pharmaceutically acceptable salt of the same.

It is another object of the present invention to provide a method for preparing the 2-phenylbenzofuran derivative above.

It is also an object of the present invention to provide a pharmaceutical composition comprising the novel 2-phenylbenzofuran derivative or the pharmaceutically acceptable salt thereof as an active ingredient for preventing or treating inflammatory disease.

It is further an object of the present invention to provide a health food composition comprising the novel 2-phenylbenzofuran derivative or the pharmaceutically acceptable salt thereof as an active ingredient for preventing or improving inflammatory disease.

To achieve the above objects, the present invention provides a compound selected from the group consisting of:
3-(7-methoxy-2-p-tolylbenzofuran-5-yl)propane-1-ol; and
4-(5-(3-hydroxypropyl)-7-methoxybenzofuran-2-yl)benzene-1,2-diol
or a pharmaceutically acceptable salt thereof.

The present invention also provides a method for preparing the 2-phenylbenzofuran derivative above.

In addition, the present invention provides a pharmaceutical composition comprising the novel 2-phenylbenzofuran derivative or the pharmaceutically acceptable salt thereof as an active ingredient for preventing or treating inflammatory disease.

Further, the present invention provides a health food composition comprising the novel 2-phenylbenzofuran derivative or the pharmaceutically acceptable salt thereof as an active ingredient for preventing or improving inflammatory disease

### ADVANTAGEOUS EFFECT

The novel 2-phenylbenzofuran derivative or the pharmaceutically acceptable salt thereof of the present invention is excellent in suppressing NO, IL-6, and TNF-alpha induced by macrophages, so that it can be effectively used for a pharmaceutical composition for preventing or treating inflammatory disease.

### DESCRIPTION OF THE PREFERRED EMBODIMENTS

Hereinafter, the present invention is described in detail.

The present invention provides a 2-phenylbenzofuran derivative selected from the group consisting of:
3-(7-methoxy-2-p-tolylbenzofuran-5-yl)propane-1-ol; and
4-(5-(3-hydroxypropyl)-7-methoxybenzofuran-2-yl)benzene-1,2-diol
or a pharmaceutically acceptable salt thereof.

The 2-phenylbenzofuran derivative of the present invention can be used as a form of a pharmaceutically acceptable salt, in which the salt is preferably acid addition salt formed by pharmaceutically acceptable free acids. The acid addition salt herein can be obtained from inorganic acids such as hydrochloric acid, nitric acid, phosphoric acid, sulfuric acid, hydrobromic acid, hydroiodic acid, nitrous acid, and phosphorous acid; non-toxic organic acids such as aliphatic mono/dicarboxylate, phenyl-substituted alkanoate, hydroxy alkanoate, alkandioate, aromatic acids,and aliphatic/aromatic sulfonic acids; or organic acids such as acetic acid, benzoic acid, citric acid, lactic acid, maleic acid, gluconic acid, methanesulfonic acid, 4-toluenesulfonic acid, tartaric acid, and fumaric acid. The pharmaceutically non-toxic salts are exemplified by sulfate, pyrosulfate, bisulfate, sulphite, bisulphite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutylate, caprate, heptanoate, propiolate, oxalate, malonate, succinate, suberate, cabacate, fumarate, maliate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutylate, citrate, lactate, hydroxybutylate, glycolate, malate, tartrate, methanesulfonate, propanesulfonate, naphthalene-1-sulfonate, naphthalene-2-sulfonate, and mandelate.

The acid addition salt in this invention can be prepared by the conventional method known to those in the art. For example, the 2-phenylbenzofuran derivative of the invention is dissolved in an organic solvent such as methanol, ethanol, acetone, methylenechloride, or acetonitrile, to which organic acid or inorganic acid is added to induce precipitation. Then, the precipitate is filtered and dried to give the salt. Or the solvent and the excessive acid are distillated under reduced pressure, and dried to give the salt. Or the precipitate is crystallized in an organic solvent to give the same.

A pharmaceutically acceptable metal salt can be prepared by using a base. Alkali metal or alkali earth metal salt is obtained by the following processes: dissolving the compound in excessive alkali metal hydroxide or alkali earth metal hydroxide solution; filtering non-soluble compound salt; evaporating the remaining solution and drying thereof. At this time, the metal salt is preferably prepared in the pharmaceutically suitable form of sodium, potassium, or calcium salt. And the corresponding silver salt is prepared by the reaction of alkali metal or alkali earth metal salt with proper silver salt (ex; silver nitrate).

The present invention includes not only the 2-phenylbenzofuran derivative of the invention, but also a pharmaceutically acceptable salt thereof, and a solvate, an optical isomer, or a hydrate possibly produced from the same.

The present invention also provides a method for preparing the 2-phenylbenzofuran derivative of the invention.

### Preparation Method 1 (not part of the claimed invention):

A method for preparing the derivative of the invention is disclosed comprising the following steps, as shown in reaction formula 1 below:
preparing the compound represented by formula 5 via Sonogashira reaction by reacting the compound represented by formula 3 with the compound represented by formula 4 in the presence of a palladium/charcoal catalyst and a base (step 1); and
preparing the compound represented by formula 1a by reducing the compound represented by formula 5 obtained in step 1 in the presence of sodiumborohydride (step 2):

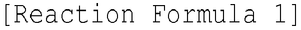

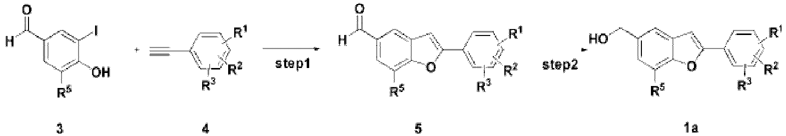

In the reaction formula 1, R¹, R², R³, and R⁵ are as defined by the compounds of the invention, and the compound represented by formula 1a is a derivative of the compound of the invention.

Hereinafter, the preparation method 1 is described in more detail step by step.

In the preparation method 1, step 1 is to give the compound represented by formula 5 by inducing Sonogashira reaction and cyclization of the compound represented by formula 3 and the compound represented by formula 4 in the presence of a palladium/charcoal catalyst and a base.

Particularly, the said Sonogashira reaction is the reaction to produce carbon-carbon bond by substituting halogen with alkyne by using a palladium catalyst via organic synthesis. And, the said cyclization is the reaction to produce a ring by adding a hydroxy group of the compound represented by formula 3 to the Alkyne substituted by Sonogashira reaction.

At this time, the palladium catalyst used for the Sonogashira reaction in step 1 is not limited and any conventional palladium catalyst acceptable for Sonogashira reaction can be used but preferably selected from the group consisting of palladium charcoal (Pd/C), tetrakistriphenylphosphinepalladium (Pd(PPh₃)₄), bistriphenylpalladiumdichloride (PdCl₂(PPh₃)₂), trisdibenzylideneacetonepalladium (Pd₂(dba)₃), 1,1-bis(diphenylphosphinoferrocene)dichloropalladium (PdCl₂(dppf)), arylpalladiumchloridedimer ([PdCl(allyl)]₂), diacetatepalladium (Pd(OAc)₂), and palladiumdichloride (PdCl₂), and more preferably palladium charcoal (Pd/C) is selected.

The base in step 1 is exemplified by such an organic acid as triethylamine, N,N-diisopropylethylamine, 1,8-diazabicyclo[5.4.0]-7-undecene (DBU), pyridine, and L-prolinol or such an inorganic base as sodium carbonate, potassium carbonate, potassium hydroxide, sodium hydroxide, cesium carbonate, and barium hydroxide, which is used by equivalent or excessive amount.

Further, the solvent used in step 1 is preferably selected form the group consisting of water, tetrahydrofuran, dioxane, 1,2-dimethoxyethane, benzene, toluene, xylene, methanol, ethanol, propanol, butanol, dimethylformamide (DMF), dimethylsulfoxide (DMSO), and acetonitrile, which is used independently or together. Among these, water is more preferred.

Also, the reaction of step 1 is preferably induced at 0°C or under the boiling point of a solvent.

In the preparation method 1 of the present invention, step 2 is to give the compound represented by formula 1a by inducing reduction of the compound represented by formula 5 obtained in step 1.

Particularly in step 2, an aldehyde group of the compound represented by formula 5 is converted into an alcohol group.

At this time, a reagent for the reduction above is not limited and any conventional reagent that is able to reduce aldehyde is accepted without limitation, but is preferably selected from the group consisting of sodiumborohydride (NaBH₄) and lithiumaluminumhydride (LiAlH₄), and sodiumborohydride (NaBH₄) is more preferred.

The solvent used in step 2 is preferably selected from the group consisting of tetrahydrofuran, dioxane, 1,2-dimethoxyethane, benzene, toluene, xylene, methanol, ethanol, propanol, butanol, dimethylformamide (DMF), dimethylsulfoxide (DMSO), and acetonitrile, which is used independently or together. Among these, tetrahydrofuran is more preferred.

Also, the reaction of step 2 is preferably induced at 0°C or under the boiling point of a solvent.

### Preparation Method 2 (not part of the claimed invention):

A method for preparing the derivative of the invention is disclosed comprising the following steps, as shown in reaction formula 2:
preparing the compound represented by formula 6 by reacting the compound represented by formula 5 with methyltriphenylphosphine halide and removing water from the same in the presence a base catalyst (step 1); and
preparing the compound represented by formula 1b by inducing hydroboration using borane or diborane and oxidation using hydrogen peroxide of the compound represented by formula 6 obtained in step 1 (step 2):

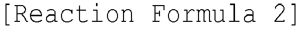

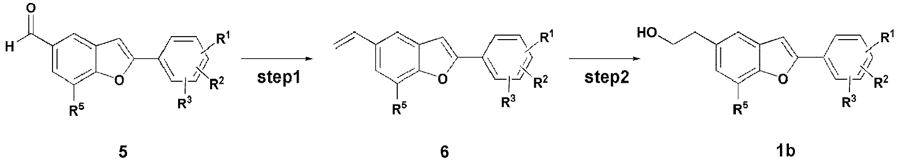

In the reaction formula 2, R¹, R², R³, and R⁵ are as defined by the compounds of the invention, and the compound represented by formula 1b is a derivative of the compound of the invention.

Hereinafter, the preparation method 2 is described in more detail step by step.

In the preparation method 2, step 1 is to give the compound represented by formula 6 by reacting the compound represented by formula 5 with methyltriphenylphosphine halide and removing water from the same in the presence a base catalyst.

Particularly, an aldehyde group of the compound represented by formula 5 is reacted with methyltriphenylphosphine halide and then water is removed in the presence of a base catalyst, resulting in the compound represented by formula 6 containing a vinyl group.

At this time, the methyltriphenylphosphine halide is preferably methyltriphenylphosphine iodide (CH₃PPh₃I).

The base in step 1 is preferably sodium hydride, potassium hydride, lithium hydride, or cesium hydride, and sodium hydride is more preferred.

The solvent in step 1 is preferably exemplified by dimethylformamide, dimethylsulfoxide, and acetonitrile.

Also, the reaction of step 2 is preferably induced at 0°C or under the boiling point of a solvent.

In the preparation method 2 of the present invention, step 2 is to give the compound represented by formula 1b by inducing hydroboration and oxidation of the compound represented by formula 6 obtained in step 1.

Particularly, step 2 is to give the compound represented by formula 1b comprising a primary alcohol group by inducing hydroboration of an alkene group of the compound represented by formula 6, and oxidation of the same.

At this time, borane (BH₃) or diborane (B₂H₆) is preferably used for the hydroboration as a boron compound, and hydrogen peroxide (H₂O₂) is preferably used for the oxidation.

The base used in step 2 is an inorganic base selected from the group consisting of sodium hydroxide, potassium hydroxide, lithium hydroxide, barium hydroxide, sodium carbonate, potassium carbonate and cesium carbonate, which is used by equivalent or excessive amount.

### Preparation method 3:

The present invention also provides a method for preparing the derivative of the invention comprising the following steps, as shown in reaction formula 3 below:
preparing the compound represented by formula 8 by reacting the compound represented by formula 5 with the compound represented by formula 7 in the presence of a base catalyst (step 1);
preparing the compound represented by formula 9 by inducing hydrogenation of the compound represented by formula 8 obtained in step 1 in the presence of an acid using palladium charcoal as a catalyst (step 2); and
preparing the compound represented by formula 1c by reducing the compound represented by formula 9 obtained in step 2 in the presence of sodium borohydride (step 3) :

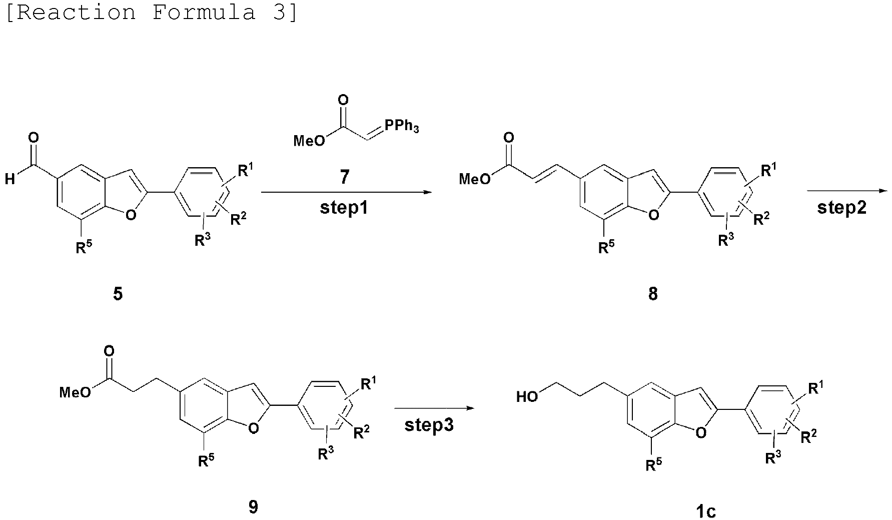

In the reaction formula 3, R¹, R², R³, and R⁵ are as defined in claim 1, and the compound represented by formula 1c is a derivative of the compound of the invention.

Hereinafter, the preparation method 3 is described in more detail step by step.

In the preparation method 3 of the present invention, step 1 is to give the compound represented by formula 8 by reacting the compound represented by formula 5 with the compound represented by formula 7.

Particularly, this step is performed by the same manner as step 1 of the preparation method 2 above except that the compound represented by formula 7 is used instead of methyltriphenylphosphine halide.

In the preparation method 3 of the present invention, step 2 is to give the compound represented by formula 9 by inducing hydrogenation of the compound represented by formula 8 obtained in step 1.

Particularly, an alkene group of the compound represented by formula 8 is hydrogenated to produce an alkyl group.

At this time, the hydrogenation is not limited and performed as generally. Preferably, the reaction is performed in the presence of an acid using palladium charcoal as a catalyst.

The solvent used in step 2 is preferably selected from the group consisting of tetrahydrofuran, 1,2-dimethoxyethane, benzene, toluene, xylene, dimethylformamide, dimethylsulfoxide, and acetonitrile, which is used independently or together. Among these, tetrahydrofuran is more preferred.

Also, the reaction of step 2 is preferably induced at 0°C or under the boiling point of a solvent.

In the preparation method 3 of the present invention, step 3 is to give the compound represented by formula 1c by reducing the compound represented by formula 9 obtained in step 2.

Particularly, this step is performed by the same manner as step 2 of the preparation method 1.

In those preparation methods 1 ∼ 3, after the reaction of each step, additional steps of extracting with an organic solvent, drying, filtering, and distillating under reduced pressure can be performed and also column chromatography or re-crystallization can be performed.

The present invention also provides a pharmaceutical composition comprising the novel 2-phenylbenzofuran derivative or the pharmaceutically acceptable salt thereof as an active ingredient for preventing or treating inflammatory disease.

The inflammatory disease herein is preferably selected from the group consisting of dermatitis, allergy, atopy, conjunctivitis, periodontitis, rhinitis, otitis media, laryngopharyngitis, tonsilitis, pneumonia, gastric ulcer, gastritis, Crohn's disease, colitis, hemorrhoid, gout, ankylosing spondylitis, rheumatic fever,

lupus, fibromyalgia, psoriatic arthritis, osteoarthritis, rheumatoid arthritis, periarthritis of shoulder, tendinitis, tenosynovitis, peritendinitis, dermatitis, hepatitis, cystitis, nephritis, Sjogren's syndrome, multiple sclerosis, and acute or chronic inflammatory disease.

Particularly, the 2-phenylbenzofuran derivative of the present invention displays an excellent cell survival rate with low cytotoxicity (experimental example 1), as high NO generation suppression rate as at least 30% by immune response (experimental example 2-1). The 2-phenylbenzofuran derivative of the invention is also excellent in suppressing the generation of IL-6 generation by immune response (experimental example 2-2) and is excellent in suppressing the generation of TNF-alpha by immune response as well (experimental example 2-3).

Therefore, the 2-phenylbenzofuran derivative of the present invention or the pharmaceutically acceptable salt thereof can be used as an active ingredient for a pharmaceutical composition for preventing or treating inflammatory disease.

The 2-phenylbenzofuran derivative of the present invention or the pharmaceutically acceptable salt thereof can be prepared for oral or parenteral administration by mixing with generally used diluents or excipients such as fillers, extenders, binders, wetting agents, disintegrating agents and surfactants.

The formulations for oral administration are exemplified by tablets, pills, powders, granules, capsules, and troches, etc. The solid formulations for oral administration are prepared by mixing one or more suitable excipients such as starch, calcium carbonate, sucrose or lactose, gelatin, etc. in addition to the 2-phenylbenzofuran derivative of the present invention or the pharmaceutically acceptable salt thereof. Except for the simple excipients, lubricants, for example magnesium stearate, talc, etc, can be used. Liquid formulations for oral administrations are suspensions, solutions, emulsions and syrups, and the above-mentioned formulations can contain various excipients such as wetting agents, sweeteners, aromatics and preservatives in addition to generally used simple diluents such as water and liquid paraffin.

Formulations for parenteral administration are sterilized aqueous solutions, water-insoluble excipients, suspensions, emulsions, lyophilized preparations, and suppositories. Water insoluble excipients and suspensions can contain, in addition to the active compound or compounds, propylene glycol, polyethylene glycol, vegetable oil like olive oil, injectable ester like ethylolate, etc. Suppositories can contain, in addition to the active compound or compounds, witepsol, macrogol, tween 61, cacao butter, laurin butter, glycerol, gelatin, etc.

The effective dosage of the 2-phenylbenzofuran derivative of the present invention or the pharmaceutically acceptable salt thereof can be determined according to age, weight, gender, administration method, health condition, and severity of disease. The dosage is 0.1 ∼ 1000 mg/day for an adult patient (70 Kg), preferably 1 ∼ 500 mg/day, which can be administered several times a day or preferably once a day or a couple of times a day.

The pharmaceutical composition of the present invention can be administered alone or treated together with surgical operation, hormone therapy, chemo-therapy and biological regulators.

The present invention also provides a health food composition comprising the novel 2-phenylbenzofuran derivative or the pharmaceutically acceptable salt thereof as an active ingredient for preventing or improving inflammatory disease.

The inflammatory disease herein is preferably selected from the group consisting of dermatitis, allergy, atopy, conjunctivitis, periodontitis, rhinitis, otitis media, laryngopharyngitis, tonsilitis, pneumonia, gastric ulcer, gastritis, Crohn's disease, colitis, hemorrhoid, gout, ankylosing spondylitis, rheumatic fever, lupus, fibromyalgia, psoriatic arthritis, osteoarthritis, rheumatoid arthritis, periarthritis of shoulder, tendinitis, tenosynovitis, peritendinitis, dermatitis, hepatitis, cystitis, nephritis, Sjogren's syndrome, multiple sclerosis, and acute or chronic inflammatory disease.

Particularly, the 2-phenylbenzofuran derivative of the present invention displays an excellent cell survival rate with low cytotoxicity (experimental example 1), as high NO generation suppression rate as at least 30% by immune response (experimental example 2-1). The 2-phenylbenzofuran derivative of the invention is also excellent in suppressing the generation of IL-6 generation by immune response (experimental example 2-2) and is excellent in suppressing the generation of TNF-alpha by immune response as well (experimental example 2-3).

Therefore, the 2-phenylbenzofuran derivative of the present invention or the pharmaceutically acceptable salt thereof can be used as an active ingredient for a health food composition for preventing or improving inflammatory disease.

The food herein is not limited. For example, the derivative of the present invention can be added to drinks, meats, sausages, breads, chocolates, candies, snacks, cookies, pizza, ramyuns, flour products, gums, dairy products including ice cream, soups, beverages, tea, drinks, alcohol drinks and vitamin complex, etc, and in wide sense, almost every food applicable in the production of health food can be included.

The 2-phenylbenzofuran derivative of the present invention or the pharmaceutically acceptable salt thereof can be used as a food additive. In that case, the 2-phenylbenzofuran derivative of the present invention or the pharmaceutically acceptable salt thereof can be added as it is or as mixed with other food components according to the conventional method. The mixing ratio of active ingredients can be regulated according to the purpose of use (prevention or improvement). In general, to produce health food or beverages, the 2-phenylbenzofuran derivative of the present invention or the pharmaceutically acceptable salt thereof is added preferably by 0.1 ∼ 90 weight part. However, if long term administration is required for health and hygiene or regulating health condition, the content can be lower than the above but higher content can be accepted as well since the 2-phenylbenzofuran derivative of the present invention or the pharmaceutically acceptable salt thereof has been proved to be very safe.

The health beverages of the present invention can additionally include various flavors or natural carbohydrates, etc, like other beverages. The natural carbohydrates above can be one of monosaccharides such as glucose and fructose, disaccharides such as maltose and sucrose, polysaccharides such as dextrin and cyclodextrin, and sugar alcohols such as xilytole, sorbitol and erythritol. Besides, natural sweetening agents (thaumatin, stevia extract, for example rebaudioside A, glycyrrhizin, etc.) and synthetic sweetening agents (saccharin, aspartame, etc.) can be included as a sweetening agent. The content of the natural carbohydrate is preferably 1 ∼ 20 g and more preferably 5 ∼ 12 g in 100 g of the composition of the present invention.

In addition to the ingredients mentioned above, the 2-phenylbenzofuran derivative of the present invention can include in variety of nutrients, vitamins, minerals (electrolytes), flavors including natural flavors and synthetic flavors, coloring agents and extenders (cheese, chocolate, etc.), pectic acid and its salts, alginic acid and its salts, organic acid, protective colloidal viscosifiers, pH regulators, stabilizers, antiseptics, glycerin, alcohols, carbonators which used to be added to soda, etc. The 2-phenylbenzofuran derivative of the present invention can also include natural fruit juice, fruit beverages and/or fruit flesh addable to vegetable beverages.

### <Preparative Example 1> 2-(3,4-dimethoxyphenyl)-7-methoxybenzofuran-5-carbaldehyde

5-iodovanillin 6 (300 mg, 1.08 mmol), 10% palladium/carbon (34 mg, 0.03 mmol), triphenylphosphine (34 mg, 0.13 mmol), copper iodide (12 mg, 0.06 mmol), triethylamine (329 mg, 3.0 mmol), and water were added in a 50 ml sealed tube containing a magnetic stirrer, followed by stirring for 1 hour with degassing using argon. 4-ethinyl-1,2-dimethoxybenzene (437 mg, 2.70 mmol) was added to the reaction mixture above, followed by degassing using argon for 15 minutes. The reaction mixture was stirred for 24 hours at a high temperature. Upon completion of the reaction, the mixture was extracted with ethyl acetate. The extract was washed with brine. The organic layer was separated and washed with water, dried over anhydrous magnesium sulfate, filtered, and concentrated under reduced pressure. The residue was purified by silica gel column chromatography (hexane:ethylacetate = 7:3) to give the target compound as a yellow solid (360 mg, 62.5%) .

¹H NMR (CDCl₃, 500 MHz) δ 10.01 (1H, s, CHO), 7.70 (1H, d, *J* = 1.0 Hz, aromaticH), 7.49 (1H, dd, *J* = 1.9 Hz, 8.3 Hz, aromaticH), 7.38 (1H, d, *J* = 1.8 Hz, aromaticH), 7.36 (1H, s, aromaticH), 6.99 (1H, s, aromaticH), 6.95 (1H, d, *J* = 8.4 Hz, aromaticH), 4.10 (3H, s, CH₃), 4.00 (3H, s, CH₃), 3.95 (3H, s, CH₃);

### <Preparative Examples 2 ∼ 18>

The compounds shown in Table 1 were prepared by the same manner as described in Preparative Example 1.

**[Table 1]**

| Preparative Example | Compound | Yield | ¹H NMR |
|---|---|---|---|
| 2 | 7-methoxy-2-(4-methoxy-2-methylphenyl) benzofuran-5-carbaldehyde | 61.9% | δ 10.02 (1H, s, CHO), 7.81 (1H, d, J = 8.4 Hz, aromaticH), 7.72 (1H, s, aromaticH), 7.37 (1H, s, aromaticH), 6.85 (3H, d, J = 10 Hz, aromaticH), 4.09 (3H, s, CH3), 3.86 (3H, s, CH₃), 2.57 (3H, s, CH₃); |
| 3 | 7-methoxy-2-m-tolylbenzofur an-5-carbaldehyde | 31.5% | δ 10.01 (1H, s, CHO), 7.72 (2H, d, *J* = 1.5 Hz, aromaticH), 7.70 (1H, d, *J* = 8.0 Hz, aromaticH), 7.38 (1H, d, *J* = 0.8 Hz, aromaticH), 7.34 (1H, d, *J* = 7.5 Hz, aromaticH), 7.21 (1H, d, *J* = 7.5 Hz, aromaticH), 7.09 (1H, s, aromaticH), 4.11 (3H, s, CH₃), 2.44 (3H, s, CH₃); |
| 4 | 7-methoxy-2-p-tolylbenzofuran-5-carbaldehyde | 38.6% | δ 10.01 (1H, s, CHO), 7.80 (2H, d, *J* = 8.0 Hz, aromaticH), 7.71 (1H, s, aromaticH), 7.37 (1H, s, aromaticH), 7.27 (2H, d, *J* = 9.6 Hz aromaticH), 7.05 (1H, s, aromaticH), 4.10 (3H, s, CH₃), 2.41 (3H, s, CH₃); |
| 5 | 7-methoxy-2-(3-methoxyphenyl)benzofuran-5-carbaldehyde | 71.8% | δ 10.01 (1H, s, CHO), 7.72 (1H, d, *J* = 1.5 Hz, aromaticH), 7.49 (1H, d, *J* = 7.5 Hz, aromaticH), 7.43 (1H, t, *J* = 2.0 Hz, aromaticH), 7.38 (2H, t, *J* = 8.0 Hz aromaticH), 7.11 (1H, s, aromaticH), 6.95 (1H, dd, *J* = 2.0 Hz, 8.0 Hz, aromaticH), 4.10 (3H, s, CH₃), 3.90 (3H, s, CH₃); |
| 6 | 2-(3,5-difluorophenyl)-7-methoxybenzofuran-5-carbaldehyde | 14.1% | δ 10.02 (1H, s, CHO), 7.74 (1H, s, aromaticH), 7.41 (3H, d, *J* =4.8 aromaticH), 7.15 (1H, s, aromaticH), 6.83 (1H, t, *J* =8.8, aromaticH), 4.10 (3H, s, CH₃) |
| 7 | 7-methoxy-2-(4-methoxyphenyl)benzofuran-5-carbaldehyde | 80% | δ 10.01 (1H, s, CHO), 7.83 (2H, d, *J* = 8.0 Hz, aromaticH), 7.69 (1H, s, aromaticH), 7.34 (1H, s, aromaticH), 6.98 (2H, d, *J* = 8.0 Hz, aromaticH) 6.96 (1H, s, aromaticH), 4.09 (3H, s, CH₃), 3.87 (3H, s, CH₃); |
| 8 | 2- (3-fluorophenyl) -7-methoxybenzof uran-5-carbaldehyde | 77.8% | δ 10.02 (1H, s, CHO), 7.74 (1H, s, aromaticH), 7.68 (1H, d, *J* = 8.0 Hz, aromaticH), 7.587.62 (1H, m, aromaticH), 7.44 (1H, q, *J* = 8 Hz, aromaticH), 7.41 (1H, d, *J* = 8 Hz, aromaticH), 7.14 (1H, s, aromaticH), 7.09 (1H, s, aromaticH),4.10 (1H, s, CH₃), 2.44 (3H, s, CH₃); |
| 9 | 2-(4-(dimethylamino)phenyl)-7-methoxybenzofuran-5-carbaldehyde | 45.8% | δ 9.99 (1H, s, CHO), 7.70 (2H, d, *J* = 8.0 Hz, aromaticH), 7.66 (1H, s, aromaticH), 7.32 (1H, s, aromaticH), 6.87 (1H, s, aromaticH), 6.76 (2H, d, *J* = 8.0 Hz, aromaticH), 4.09 (3H, s, CH₃), 3.03 (6H, s, CH₃); |
| 10 | 7-methoxy-2-(2-methoxyphenyl )benzofuran-5-carbaldehyde | 69.1% | δ 10.01 (1H, s, CHO), 8.13 (1H, dd, J = 1.6, 1.6 Hz, aromaticH), 7.73 (1H, d, J = 1.6 Hz, aromaticH), 7.43 (1H, s, aromaticH), 7.34-7.39 (2H, m, aromaticH), 7.077.11 (1H, m, aromaticH), 7.03 (1H, d, J = 8.4 Hz, aromaticH), 4.10 (3H, s, CH3), 4.02 (3H, s, CH3); |
| 11 | 7-methoxy-2-(3-(trifluoromethyl)phenyl)benzofuran-5-carbaldehyde | 52.1% | δ 10.02 (1H, s, CHO), 8.14 (1H, s, aromaticH), 8.07(1H, d, *J* = 7.2 Hz, aromaticH), 7.75 (1H, d, *J* = 1.6 Hz, aromaticH), 7.62 (1H, q, *J* = 8 Hz aromaticH), *J* = 7.41 (1H, d, *J* = 1.2 Hz, aromaticH), 7.21 (1H, s, aromaticH), 4.11 (3H, s, CH₃); |
| 12 | 2-(3,5-dimethoxyphenyl)-7-methoxybenzofuran-5-carbaldehyde | 53.4% | δ 10.01 (1H, s, CHO), 7.72 (1H, d, *J* = 0.8 Hz, aromaticH), 7.38 (1H, s, aromaticH), 7.09 (1H, s, aromaticH), 7.04 (2H, d, *J* = 2.8 Hz, aromaticH), 6.51 (2H, t, *J =* 2.0 Hz, aromaticH), 4.10 (3H, s, CH₃), 3.88 (6H, s, CH₃). |
| 13 | 7-methoxy-2-(4-(trifluoromethyl)phenyl)benzofuran-5-carbaldehyde | 73.0% | δ 10.02 (1H, s, CHO), 8.01 (1H, d, *J* = 8.4 , aromaticH), 7.75 (1H, d, *J* = 0.8 Hz, aromaticH), 7.72 (2H, d, *J* = 8.4 , aromaticH), 7.41 (1H, d, *J* = 1.2 Hz aromaticH), 7.23 (1H, s, aromaticH), 4.11 (3H, s,CH₃); |
| 14 | 2-(4-(tert-butyldimethyl siloxy)-3-methoxyphenyl)-7-methoxybenzofuran-5-carbaldehyde | 61.9% | δ 10.00 (1H, s, CHO), 7.69 (1H, d, *J* = 1.6 Hz, aromatic-H), 7.35-7.39 (3H, m, aromatic-H), 6.97 (1H, s, aromatic-H), 6.92 (1H, dd, *J*₁ = 0.8 Hz, *J*₂ = 1.2 Hz, aromatic-H), 4.09 (3H, s, CH₃), 3.92 (3H, s, CH₃), 1.01 (9H, t, *J* = 3.2 Hz, CH₃), 0.12 (6H, t, *J* = 3.2 Hz, CH₃); |
| 15 | 2-(3-(tert-butyldimethyl siloxy)-4-methoxyphenyl)-7-methoxybenzofuran-5-carbaldehyde | 75.2% | δ 10.00 (1H, s, CHO), 7.68 (1H, d, *J* = 1.2 Hz, aromatic-H), 7.49 (1H, dd, *J*₁ = 2.0 Hz, *J*₂ = 2.0 Hz, aromatic-H), 7.34 (2H, t, *J* = 1.6 Hz, aromatic-H), 6.93 (1H, s, aromatic-H), 6.92 (1H, d, *J* = 8.8 Hz, aromatic-H), 4.09 (3H, s, CH₃), 3.86 (3H, s, CH₃), 1.03 (9H, t, *J* = 2.8 Hz, CH₃), 0.20 (6H, t, *J* = 3.2 Hz, CH₃); |
| 16 | 2-(3,5-bis(tert-butyldimethyl silyloxy)phenyl)-7-methoxybenzofuran-5-carbaldehyde | 88.2% | δ10.00 (1H, s, CHO), 7.70 (1H, d, *J* = 1.2 Hz, aromatic-H), 7.36 (1H, d, *J* = 1.2 Hz, aromatic-H), 7.03 (1H, s, aromatic-H), 6.99 (2H, d , *J* = 2.0 Hz, aromatic-H), 6.36 (1H, t, *J* = 2.0 Hz, aromatic-H), 4.10 (3H, s, CH₃), 1.01 (18H, t, *J* = 3.2 Hz, CH₃), 0.24 (12H, t, *J* = 3.2 Hz, CH₃); |
| 17 | 2-(3,4-bis(tert-butyldimethyl silyloxy)phenyl)-7-methoxybenzofuran-5-carbaldehyde | 73.1% | δ 10.00 (1H, s, CHO), 7.68 (1H, s, aromatic-H), 7.39 (1H, dd, *J*₁ = 2.0 Hz, *J*₂ = 2.4 Hz, aromatic-H), 7.34 (1H, s, aromatic-H), 7.32 (1H, d , *J* = 2.0 Hz, aromatic-H), 6.89-6.91 (2H, m, aromatic-H), 4.09 (3H, s, CH₃), 0.99-1.03 (18H, m, CH₃), 0.23-0.26 (12H, m, CH₃); |
| 18 | 2-(4-(tert-butyldimethyl silyloxy)-3,5-dimethoxyphenyl)-7-methoxybenzofuran-5-carbaldehyde | 90.4% | δ 10.00 (1H, s, CHO), 7.69 (1H, d, *J* = 1.2 Hz, aromatic-H), 7.36 (1H, d, *J* = 1.2 Hz, aromatic-H), 7.07 (1H, s, aromatic-H), 6.99 (1H, s, aromatic-H), 4.09 (3H, s, CH₃), 3.90 (6H, s, CH₃), 1.02 (9H, t, *J* = 2.8 Hz, CH₃), 0.16 (6H, t, *J* = 2.8 Hz, CH₃); |

### <Preparative Example 19> (E)-methyl-3-[2-(3,4-dimethoxyphenyl)-7-methoxybenzofuran-5-yl]acrylate

2-(3,4-dimethoxyphenyl)-7-methoxybenzofuran-5-carbaldehyde (100 mg, 0.32 mmol) prepared in Preparative Example 1 was dissolved in dichloromethane (5 mL), to which methyl(triphenylphosphoranyliden)acetate (1.07g, 3.20 mmol) was added, followed by stirring at a high temperature for 12 hours. Upon completion of the reaction, the reaction mixture was concentrated under reduced pressure. The residue was purified by silica gel column chromatography (hexane:ethylacetate = 8:2) to give the target compound as a white solid (100 mg, 85.5%).

¹H NMR (CDCl₃, 500 MHz) δ 7.77 (1H, d, *J* = 15.5 Hz, =CH), 7.47 (1H, dd, *J* = 1.0 Hz, 8.0 Hz, aromaticH), 7.37 (2H, d, *J* = 1.0 Hz, aromaticH), 6.97 (1H, s, aromaticH), 6.94 (1H, d, *J* = 8.5 Hz, aromaticH), 6.90 (1H, s, aromaticH), 6.42 (1H, d, *J* = 15.5 Hz, =CH), 4.08 (3H, s, CH₃), 3.99 (3H, s, CH₃), 3.94 (3H, s, CH₃), 3.83 (3H, s, CH₃);

### <Preparative Examples 20 ∼ 36>

The compounds shown in Table 2 were prepared by the same manner as described in Preparative Example 19.

**[Table 2]**

| Preparative Example | Compound | Yield | ¹H NMR |
|---|---|---|---|
| 20 | (E)-methyl-3-(7-methoxy-2-(4-methoxy-2-methylphenyl)benzofuran-5-yl)acrylate | 90.2% | δ 7.78 (2H, t, *J* = 8.4 Hz, aromaticH), 7.35 (1H, s, aromaticH), 6.97 (1H, s, aromaticH), 6.84 (2H, d, *J* = 6.8 Hz, aromaticH),6.77 (1H, s, aromatic H), 6.42 (1H, d, *J* = 16.0 Hz, =CH), 4.07 (3H, s, CH₃), 3.85 (3H, s, CH₃), 3.82 (3H, s, CH₃), 2.55 (3H, s, CH₃); |
| 21 | (E)-methyl-3-(7-methoxy-2-m-tolylbenzofuran-5-yl)acrylate | 83% | δ 7.78 (1H, d, *J* = 16.0 Hz, =CH), 7.70 (2H, d, *J* = 17.6 Hz, aromaticH), 7.36 (2H, s, aromaticH), 7.20 (1H, s, aromaticH), 7.36 (2H, s, aromaticH), 6.42 (1H, d, *J* = 16.0 Hz, =CH), 4.08 (3H, s, CH₃), 3.82 (3H, s, CH₃), 2.42 (3H, s, CH₃); |
| 22 | (E)-methyl-3-(7-methoxy-2-p-tolylbenzofuran-5-yl)acrylate | 85% | δ 7.77 (1H, d, *J* = 16.0 Hz, =CH), 7.76 (1H, d, *J* = 8.4 Hz, aromaticH), 7.34 (1H, s, aromaticH), 6.96 (2H, d, *J* = 6.4 Hz, aromaticH), 6.42 (1H, d, *J* = 16.0 Hz, =CH), 4.08 (3H, s, CH₃), 3.82 (3H, s, CH₃), 2.40 (3H, s, CH₃); |
| 23 | (E)-methyl-3-(7-methoxy-2-(3-methoxyphenyl) benzofuran-5-yl)acrylate | 57.6% | δ 7.77 (1H, d, *J* = 16.0 Hz, =CH), 7.47 (1H, d, *J* = 7.5 Hz, aromaticH), 7.37-7.41(3H, m, aromaticH), 6.98 (2H, d, *J* = 1.0 Hz, aromaticH), 6.92 (1H, dd, *J* = 2.0 Hz, 8.5 Hz, aromaticH),6.42 (1H, d, *J* = 16.0 Hz, =CH), 4.08 (3H, s, CH3), 3.89 (3H, s, CH3), 3.82 (3H, s, CH3); |
| 24 | (E)-methyl-3-(2-(3,5-difluorophenyl)-7-methoxybenzofuran-5-yl)acrylate | 28.9% | δ 7.77 (1H, d, *J* = 16.0 Hz, =CH), 7.367.39 (3H, m, aromaticH), 7.05 (1H, s, aromaticH), 7.01 (1H, d, *J* = 1.0 Hz, aromaticH), 6.81(1H, t, *J* = 2.0 Hz, 8.5Hz aromaticH), 6.43 (1H, d, *J* = 16.0 Hz, =CH), 4.08 (3H, s, CH3), 3.83 (3H, s, CH3) |
| 25 | (E)-methyl-3-(7-methoxy-2-(4-methoxyphenyl) benzofuran-5-yl)acrylate | 60.7% | δ 7.82 (1H, s, aromaticH), 7.80 (1H, s, aromaticH), 7.76 (1H, d, *J* = 16.0 Hz, =CH), 7.32 (1H, s, aromaticH), 6.98 (1H, s, aromaticH), 6.96 (1H, s, aromaticH), 6.41 (1H, d, *J* = 16.0 Hz, =CH), 4.07 (3H, s, CH₃), 3.86 (3H, s, CH₃), 3.82 (3H, s, CH₃); |
| 26 | (E)-methyl-3-(2-(3-fluorophenyl)-7-methoxybenzofuran-5-yl)acrylate | 88.7% | δ 7.77 (1H, d, *J* = 16.0 Hz, =CH), 7.647.66 (1H, m, aromaticH), 7.567.59 (H, m, aromaticH), 7.387.44 (1H, m, aromaticH), 7.36 (1H, s, aromaticH), 7.06-7.08 (1H, m, aromaticH), 7.03 (1H, s, aromaticH), 7.00 (1H, s, aromaticH), 6.42 (1H, d, *J* = 16.0 Hz, =CH), 4.08 (3H, s, CH₃), 3.82 (3H, s, CH₃); |
| 27 | (E)-methyl-3-(2-(4-dimethylamino) phenyl)-7-methoxybenzofuran-5-yl)acrylate | 79.2% | δ 7.76 (1H, d, *J* = 16.0 Hz, =CH),, 7.70 (2H, d, *J* = 8.0 Hz aromaticH), 7.30 (1H, s, aromaticH), 6.93 (1H, s, aromaticH), 6.79 (3H, s, aromaticH), 6.40 (1H, d, *J* = 16.0 Hz, =CH), 4.07 (3H, s, CH3), 3.03 (6H, s, CH₃); |
| 28 | (E)-methyl-3-(7-methoxy-2-(2-methoxyphenyl) benzofuran-5-yl)acrylate | 53.8% | δ 8.10 (1H, dd, *J* = 2.0, 2.0 Hz, aromaticH), 7.77 (1H, d, *J* = 15.6 Hz, =CH),, 7.36 (2H, d, *J* = 1.6 Hz aromaticH), 7.33 (2H, dd, *J* = 2.8, 1.6 Hz, aromaticH), 7.057.09 (1H, m, aromaticH), 7.01 (1H, d, *J* = 10.4 aromaticH), 6.97 (1H, d, *J* = 1.6 aromaticH), 6.41 (1H, d, *J* = 16.0 Hz, =CH), 4.07 (3H, s, CH₃), 4.01 (3H, s, CH₃), 3.82 (3H, s, CH₃); |
| 29 | (E)-methyl-3-(7-methoxy-2-(3-(trifluoromethyl)phenyl)benzofuran-5-yl)acrylate | 86.3% | 58.12 (1H, s, aromaticH), 7.77 (1H, d, *J* = 15.6 Hz, =CH), 7.59 (2H, t, *J* = 7.6 Hz aromaticH), 7.37 (1H, d, *J* = 1.2 Hz, aromaticH), 7.11 (1H, s, aromaticH), 7.01 (1H, d, *J* = 0.8 aromaticH),6.43 (1H, d, *J* = 15.6 Hz, =CH), 4.08 (3H, s, CH₃), 3.83 (3H, s, CH₃); |
| 30 | (E)-methyl-3-(2-(3,5-dimethoxyphenyl)-7-methoxybenzofuran-5-yl)acrylate | 58.5% | δ7.77 (1H, d, *J* = 16 Hz, =CH), 7.35 (1H, s, aromaticH), 7.03 (2H, t, *J* = 1.2, aromaticH), 6.99 (2H, d, *J* = 8.4 Hz, aromaticH), 6.49 (1H, t, *J* = 2, aromaticH), 6.42 (1H, d, *J* = 15.5 Hz, =CH), 4.07 (3H, s, CH₃), 3.87 (6H, s, CH₃), 3.82 (3H, s, CH₃) |
| 31 | (E)-methyl-3-(7-methoxy-2-(4-(trifluoromethyl)phenyl)benzofuran-5-yl)acrylate | 87.2% | δ7.98 (2H, d, *J* = 8.0 Hz aromaticH), 7.77 (1H, d, *J* = 16.0 Hz, =CH), 7.70 (2H, t, *J* = 8 Hz aromaticH), 7.38 (1H, d, *J* = 1.6 Hz, aromaticH), 7.13 (1H, s, aromaticH), 7.02 (1H, d, *J* = 1.2 aromaticH), 6.43 (1H, d, *J* = 15.6 Hz, =CH), 4.08 (3H, s, CH3), 3.83 (3H, s, CH3); |
| 32 | (E)-methyl-3-(2-4(tert-butyldimethylsilyloxy)-3-methoxyphenyl) -7-methoxybenzofuran-5-yl)acrylate | 67% | δ 7.76 (1H, d, *J* = 16.0 Hz, =CH), 7.32-7.36 (3H, m, aromatic-H), 6.96 (1H, d, *J* = 1.6 Hz aromatic-H), 6.91 (1H, d, *J* = 8.8 Hz, aromatic-H), 6.88 (1H, s, aromatic H), 6.41 (1H, d, *J* = 16.0 Hz, =CH), 4.07 (3H, s, CH₃), 3.91 (3H, s, CH₃), 3.82 (3H, s, CH₃), 1.01 (9H, t, *J* = 3.2 Hz, CH₃), 0.18 (6H, t, *J* = 3.2 Hz, CH₃); |
| 33 | (E)-methyl- 3-(2-(3-(tert-butyldimethlysilyloxy)-4-methoxyphenyl) -7-methoxybenzofuran-5-yl)acrylate | 81.9% | 5 7.76 (1H, d, *J* = 16.0 Hz, =CH), 7.47 (1H, dd, *J*₁ = 2.0 Hz, *J*₂ = 2.0 Hz, aromatic-H), 7.32 (1H, d, *J* = 2.4 Hz aromatic-H), 7.31 (1H, d, *J* = 1.2 Hz, aromatic-H), 6.95 (1H, d, *J* = 1.2 Hz, aromatic-H), 6.91 (1H, d, *J* = 8.8 Hz aromatic-H), 6.84 (1H, s, aromatic H), 6.41 (1H, d, *J* = 16.0 Hz, =CH), 4.07 (3H, s, CH₃), 3.85 (3H, s, CH₃), 3.82 (3H, s, CH₃), 1.03 (9H, t, *J* = 2.8 Hz, CH₃), 0.20 (6H, t, *J* = 3.2 Hz, CH₃); |
| 34 | (E)-methyl- 3-(2-(3,5-bis(tert-butyldimethylsilyloxy)phenyl)-7-methoxybenzofuran-5-yl)acrylate | 84.2% | δ 7.76 (1H, d, *J* = 16.0 Hz, =CH), 7.33 (1H, d, *J* = 0.8 H, aromatic-H), 6.97 (3H, d, *J* = 2.0 Hz, aromatic-H), 6.94 (1H, s, aromatic-H), 6.41 (1H, d, *J* = 16.0 Hz, =CH), 6.35 (1H, t, *J* = 2.0 Hz, aromatic-H), 4.08 (3H, s, CH₃), 3.82 (3H, s, CH₃), 1.00 (18H, t, *J* = 2.8 Hz, CH₃), 0.24 (12H, t, *J* = 3.2 Hz, CH₃); |
| 35 | (E)-methyl-3-(2-(3,4-bis(tert-butyldimethylsilyloxy)phenyl )-7-methoxybenzofuran-5-yl)acrylate | 77.8% | 57.76 (1H, d, *J* = 16.0 Hz, =CH), 7.37 (1H, dd, *J* = 1.6 H, *J* = 2.0 H, aromatic-H), 7.31 (1H, d, *J* = 1.2 Hz aromatic-H), 7.30 (1H, d, *J* = 2.0 H, aromatic-H), 6.95 (1H, d, *J* = 1.6 H, aromatic-H), 6.89 (1H, d, *J* = 8.4 H, aromatic-H), 6.81 (1H, s, aromatic-H), 6.41 (1H, d, *J* = 16.4 Hz, =CH), 6.35 (1H, t, *J* = 2.0 Hz, aromatic-H), 4.07 (3H, s, CH₃), 3.82 (3H, s, CH₃), 0.99-1.03 (18H, m, CH₃), 0.22-0.25 (12H, m, CH₃); |
| 36 | (E)-methyl- 3-(2-(4-(tert-butyldimethylsilyloxy)-3,5-dimethoxyphenyl)-7-methoxybenzofuran-5-yl)acrylate | 66.3% | δ 7.77 (1H, d, *J* = 16.0 Hz, =CH), 7.32 (1H, d, *J* = 1.2 H, aromatic-H), 7.05 (2H, s, aromatic-H), 6.96 (1H, d, *J* = 1.2 H, aromatic-H), 6.89 (1H, s, aromatic-H),6.42 (1H, d, *J* = 16.0 Hz, =CH), 4.07 (3H, s, CH₃), 3.89 (6H, s, CH₃), 3.82 (3H, s, CH₃), 1.02 (9H, d, *J* = 3.2 Hz, CH₃), 0.16 (6H, t, *J* = 3.2 Hz, CH₃); |

### <Preparative Example 37> methyl 3-(2-(3,4-dimethoxyphenyl)-7-methoxybenzofuran-5-yl)propanoate

(E)-methyl-3-[2-(3,4-dimethoxyphenyl)-7-methoxybenzofuran-5-yl]acrylate (50 mg, 0.14 mmol) prepared in Preparative Example 14 was dissolved in tetrahydrofuran (5 mL), to which palladium/carbon (29 mg, 0.014 mmol, 10 wt%) was added with a couple of drops of acetic acid, followed by stirring at room temperature for 30 minutes. Upon completion of the reaction, the mixture was concentrated under reduced pressure. The residue was purified by silica gel column chromatography (hexane:ethylacetate = 7:3) to give the target compound as a white solid (48 mg, 95.4%) .

¹H NMR (CDCl₃, 400 MHz) δ 7.45 (1H, dd, *J* = 2.0 Hz, *J* = 2.0 Hz, aromaticH), 7.36 (1H, d, *J* = 2.0 Hz, aromaticH), 6.98 (1H, s, aromaticH), 6.92 (1H, d, *J* = 8.0 Hz, aromaticH), 6.63 (1H, d, *J* = 1.2, aromaticH), 4.06 (3H, s, CH₃), 3.94 (3H, s, CH₃), 3.91 (3H, s, CH₃), 3.68 (3H, s, CH₃), 3.02 (2H, t, *J* = 8, CH₂), 2.68 (2H, t, *J* = 8, CH₂);

### <Preparative Example 38 ∼ 54>

The compounds shown in Table 3 were prepared by the same manner as described in Preparative Example 37.

**[Table 3]**

| Preparative Example | Compound | Yield | ¹H NMR |
|---|---|---|---|
| 38 | methyl-3-(7-methoxy-2-(4-methoxy-2-methylphenyl)ben zofuran-5-yl)propanoate | 98.8% | δ7.77 (1H, d, *J* = 9. 6 Hz, aromaticH), 7.00 (1H, s, aromaticH), 6.84 (2H, t, *J* = 9.6 Hz, aromaticH), 6.72 (1H, s, aromaticH), 6.64 (1H, s, aromaticH), 4.03 (3H, s, CH₃), 3.84 (3H, s, CH₃), 3.69 (3H, s, CH₃), 4.02 (2H, t, *J* = 8 Hz, ). 2.68(2H, t, *J* = 8 Hz, CH₂), 2.54(3H, s, CH₃); |
| 39 | methyl-3-(7-methoxy-2-m-tolylbenzofuran-5-yl)propanoate | 99% | δ7.70 (1H, s, aromaticH), 7.66 (2H, d, *J* = 8 Hz, aromaticH), 7.31 (1H, t, *J* = 8 Hz, aromaticH), 7.15 (1H, d, *J* = 8 Hz, aromaticH) 6.99 (1H, s, aromaticH), 6.93 (1H, s, aromaticH), 4.04 (3H, s, CH₃), 3.02 (2H, t, *J* = 8 Hz, CH₂), 2.68 (2H, t, *J* = 8 Hz, CH₂), 2.41(3H, CH₃); |
| 40 | methyl-3-(7-methoxy-2-p-tolylbenzofuran-5-yl)propanoate | 100% | 57.76 (2H, d, *J* = 8 Hz aromaticH), 7.23 (2H, d, *J* = 8 Hz aromaticH), 6.98 (1H, s, aromaticH), 6.89 (1H, s, aromaticH) 6.63 (1H, s, aromaticH) 4.03 (3H, s, CH₃), 3.68 (3H, s, CH₃), 3.02 (2H, t, *J* = 8 Hz, CH₂), 2.68 (2H, t, *J* = 8 Hz, CH₂), 2.38(3H, s, CH₃); |
| 41 | methyl-3-(7-methoxy-2-(3-methoxyphenyl)be nzofuran-5-yl)propanoate | 89.5% | δ7.46 (2H, d, *J* = 8 Hz, aromaticH), 7.40 (1H, s, aromaticH), 7.34 (1H, t, J = 8 Hz, aromaticH), 7.00 (1H, s, aromaticH), 6.94 (1H, s, aromaticH) 6.89 (1H, q, J = 8 Hz, aromaticH), 6.65 (1H, s, aromaticH), 4.03 (3H, s, CH3), 3.88 (3H, s, CH3),). 2.38(3H, s, CH₃), 3.02 (2H, t, *J* = 8 Hz, CH₂), 2.68 (2H, t, *J* =*8 Hz, CH2*); |
| 42 | methyl-3-(2-(3,5-difluorophenyl)-7-methoxybenzofura n-5-yl)propanoate | 89.5% | δ7.34-7.38 (2H, m, aromaticH), 7.01 (2H, d, *J* = 1.2 Hz, aromaticH), 6.98 (1H, s, aromaticH), 6.756.80 (1H, m, aromaticH), 6.68 (1H, d, *J* = 1.6 Hz, aromaticH,) 4.03 (3H, s, CH₃), 3.68 (3H, s, CH₃), 3.03 (2H, t, *J* = 8 Hz, CH₂), 2.68 (2H, t, *J* = 8 Hz, CH₂); |
| 43 | methyl-3-(7-methoxy-2-(4-methoxyphenyl)be nzofuran-5-yl)propanoate | 87.9% | δ7.80 (2H, q, *J* = 4 Hz aromaticH), 7.65-6.98 (2H, m, aromaticH), 6.81 (1H, s, aromaticH), 6.62 (1H, s, aromaticH), 4.03 (3H, s, CH₃), 3.85 (3H, s, CH₃), 3.68 (3H, s, CH₃), 3.02 (2H, t, *J* = 8 Hz, CH₂), 2.68 (2H, t, *J* = 8 Hz, CH₂ ); |
| 44 | methyl-3-(2-(3-fluorophenyl)-7-methoxybenzofura n-5-yl)propanoate | 80.3% | δ7.64 (1H, d, *J* = 8 Hz, aromaticH), 7.547.58 (1H, m, aromaticH), 7.367.41 (1H, m, aromaticH), 7.04 (1H, q, *J* = 4 Hz, aromaticH) 7.01 (1H, s, aromaticH), 6.97 (1H, s, aromaticH), 6.67 (1H, d, *J* = 4 Hz, aromaticH,) 4.03 (3H, s, CH₃), 3.68 (3H, s, CH₃), 3.02 (2H, t, *J* = 8 Hz, CH₂), 2.68 (2H, t, *J* = 8 Hz, CH₂); |
| 45 | methyl-3-(2-(4-(dimethylamino)p henyl)-7-methoxybenzofura n-5-yl)propanoate | 67.6% | δ7.74 (2H, d, *J* = 8 Hz aromaticH), 6.94 (1H, s, aromaticH), 6.75 (2H, d, *J* = 8 Hz aromaticH), 6.72 (1H, s, aromaticH), 6.59 (1H, s, aromaticH), 4.03 (3H, s, CH₃), 3.68 (3H, s, CH₃), 3.01 (2H, t, *J* = 8 Hz, CH₂), 3.01 (6H, s, CH₃), 2.67 (2H, t, *J* = 8 Hz, CH₂); |
| 46 | methyl-3-(7-methoxy-2-(2-methoxyphenyl)be nzofuran-5-yl)propanoate | 86.6% | δ8.10 (1H, dd, *J* = 1.6, 1.6 Hz aromaticH), 7.297.34 (1H, m, aromaticH), 7.28 (1H, s, aromaticH), 7.06 (1H, t, *J* = 7.6 Hz aromaticH), 7.00 (2H, d, *J* = 8.4 Hz aromaticH), 6.64 (1H, d, *J* = 1.2 Hz aromaticH), 4.04 (3H, s, CH₃), 4.00 (3H, s, CH₃), 3.68 (3H, s, CH₃), 3.02 (2H, t, *J* = 8 Hz, CH₂), 2.68 (2H, t, *J* = 8 Hz, CH₂); |
| 47 | methyl-3-(7-methoxy-2-(3-(trifluoromethyl )phenyl)benzofur an-5-yl)propanoate | 68.2% | δ8.10 (1H, dd, *J* = 1.6, 1.6 Hz aromaticH), 8.03 (1H, d, *J* = 7.2 Hz aromaticH), 7.57 (1H, t, *J* = 7.6 Hz aromaticH), 7.04 (1H, s, aromaticH), 7.02 (1H, d, *J* = 0.08 Hz aromaticH), 6.68 (1H, s, aromaticH), 4.04 (3H, s, CH₃), 3.69 (3H, s, CH₃), 3.03 (2H, t, *J* = 8 Hz, CH₂), 2.69 (2H, t, *J* = 8 Hz, CH₂); |
| 48 | methyl-3-(2-(3,5-dimethoxyphenyl) -7-methoxybenzofura n-5-yl)propanoate | 87.2% | δ7.02 (2H, d, *J* = 2.4 Hz, aromaticH), 7.00 (1H, s, aromaticH), 6.93 (1H, s, aromaticH), 6.65 (1H, d, *J* = 1.2 Hz, aromaticH), 6.46 (1H, t, *J* = 2.4, aromaticH), 4.03 (3H, s, CH₃), 3.86 (6H, s, CH₃), 3.68 (3H, s, CH₃), 3.02 (2H, t, *J* = 8, CH₂), 2.68 (2H, t, *J* = 8, CH₂); |
| 49 | methyl-3-(7-methoxy-2-(4-(trifluoromethyl )phenyl)benzofur an-5-yl)propanoate | 68.2% | δ7.97 (2H, d, *J* = 8.4 Hz, aromaticH), 7.68 (2H, d, *J* = 8.4 Hz, aromaticH), 7.07 (1H, s, aromaticH), 7.03 (1H, d, *J* = 1.2 Hz, aromaticH), 6.69 (1H, d, *J* = 1.2 Hz aromaticH), 4.04 (3H, s, CH₃), 3.68 (3H, s, CH₃), 3.03 (2H, t, *J* = 8 Hz, CH₂), 2.69 (2H, t, *J* = 8 Hz, CH₂); |
| 50 | methyl-3-(2-(4-(tert-butyldimethylsil yloxy)-3-methoxyphenyl)-7-methoxybenzofura n-5-yl)propanoate | 96.4% | δ 7.32-7.34 (2H, m, aromatic-H), 6.97 (1H, d, *J* = 0.8 Hz, aromatic-H), 6.89 (1H, d, *J* = 8.0 Hz, aromatic-H), 6.81 (1H, s, aromatic-H), 6.21 (1H, d, *J* = 1.2 Hz, aromatic-H), 4.03 (3H, s, CH₃), 3.90 (3H, s, CH₃), 3.68 (3H, s, CH₃), 3.01 (2H, t, *J* = 8.4 Hz, -CH₂). 2.68 (2H, t, *J* = 7.6 Hz, CH₂), 2.54 (3H, s, CH₃), 1.00 (9H, t, *J* = 3.2 Hz, CH₃), 0.18 (6H, t, *J* = 2.8 Hz, CH₃); |
| 51 | methyl-3-(2-(3-(tert-butyldimethylsil yloxy)-4-methoxyphenyl)-7-methoxybenzofura n-5-yl)propanoate | 90.5% | δ 7.45 (1H, dd, *J*₁ = 2.0 Hz, *J*₂ = 2.4 Hz, aromatic-H), 7.31 (1H, d, *J* = 2.4 Hz, aromatic-H), 6.96 (1H, d, *J* = 0.8 Hz, aromatic-H), 6.89 (1H, d, *J* = 8.4 Hz, aromatic-H), 6.61 (1H, d, *J* = 1.2 Hz, aromatic-H), 4.03 (3H, s, CH₃), 3.84 (3H, s, CH₃), 3.68 (3H, s, CH₃), 3.01 (2H, t, *J* = 8 Hz, -CH₂). 2.67 (2H, t, *J* = 8 Hz, CH₂), 1.02 (9H, t, *J* = 2.8 Hz, CH₃), 0.19 (6H, t, *J* = 3.2 Hz, CH₃); |
| 52 | methyl-3-(2-(3,5-bis(tert-butyldimethylsil oxy)phenyl)-7-methoxybenzofura n-5-yl)propanoate | 90.4% | δ 6.98 (1H, s, aromatic-H), 6.96 (2H, d, *J* = 1.6 Hz, aromatic-H), 6.63 (1H, s, aromatic-H), 6.32 (1H, t, *J* = 2.0 Hz, aromatic-H), 4.04 (3H, s, CH₃), 3.68 (3H, s, CH₃), 3.01 (2H, t, *J* = 8.0 Hz,-CH₂). 2.68 (2H, t, *J* = 8.0 Hz, CH₂), 1.00 (18H, d, *J* = 2.4 Hz, CH₃), 0.23 (12H, t, *J* = 3.2 Hz, CH₃); |
| 53 | methyl-3-(2-(3,4-bis(tert-butyldimethylsil oxy)phenyl)-7-methoxybenzofura n-5-yl)propanoate | 83.3% | δ 7.35 (1H, dd, *J* = 2.0 Hz, *J* = 2.4 Hz, aromatic-H), 7.28 (1H, d, *J* = 2.0 Hz, aromatic-H), 6.96 (1H, d, *J* = 1.2 Hz, aromatic-H), 6.32 (1H, d, *J* = 8.4 Hz, aromatic-H), 6.75 (1H, s, aromatic-H), 6.61 (1H, d, *J* = 1.2 Hz, aromatic-H), 4.04 (3H, s, CH₃), 3.68 (3H, s, CH₃), 3.01 (2H, t, *J* = 8.0 Hz, -CH₂ ). 2.67 (2H, t, *J* = 8.0 Hz, CH₂), 0.94-1.02 (18H, m, CH₃), 0.21-0.25 (12H, m, CH₃); |
| 54 | methyl-3-(2-(4-(tert-butyldimethylsil oxy)-3,5-dimethoxyphenyl) -7-methoxybenzofura n-5-yl)propanoate | 79.2% | δ 6.96 (2H, s, aromatic-H), 6.97 (1H, d, *J* = 0.8 Hz, aromatic-H), 6.83 (1H, s, aromatic-H), 6.62 (1H, d, *J* = 1.2 Hz, aromatic-H), 4.02 (3H, s, CH₃), 3.88 (6H, s, CH₃), 3.68 (3H, s, CH₃), 3.02 (2H, t, *J* = 8.0 Hz, -CH₂ ). 2.68 (2H, t, *J* = 8.0 Hz, CH₂), 1.01 (9H, t, *J* = 2.4 Hz, CH₃), 0.15 (6H, t, *J* = 3.2 Hz, CH₃); |

### <Preparative Example 55> methyl-3-(3-(3,4-dimethoxyphenethyl)-4-hydroxy-5-methoxyphenyl)propanoate

(E)-methyl-3-[2-(3,4-dimethoxyphenyl)-7-methoxybenzofuran-5-yl]acrylate prepared in Preparative Example 14 was dissolved in tetrahydrofuran (5 mL), to which palladium/carbon (0.5 equivalent, 5 weight%) was added, followed by stirring at room temperature in the presence of hydrogen for 10 hours. Upon completion of the reaction, the mixture was filtered with celite and concentrated under reduced pressure. The residue was purified by silica gel column chromatography (hexane:ethylacetate = 7:3) to give the target compound as a white solid.

¹H NMR (CDCl₃, 400 MHz) δ 6.79 (1H, d, J = 8.0 Hz, aromaticH), 6.75 (1H, d, J = 8.0 Hz, aromaticH), 6.72 (1H, d, J = 2.0 Hz, aromaticH), 6.58 (1H, d, J = 2.0 Hz, aromaticH), 6.53 (1H, d, J = 1.6 Hz, aromaticH), 5.58 (1H, s, OH), 3.87 (3H, s, CH₃), 3.86 (3H, s, CH₃), 3.85 (3H, s, CH₃), 3.67 (3H, s,CH₃), 2.82-2.89 (6H, m, CH₂), 2.57 (2H, t, J = 8.0 Hz, CH₂).

### <Preparative Example 56> methyl-3-(4-hydroxy-3-methoxy-5-(3-methylpenethyl)phenyl)propanoate

The target compound was obtained as a white solid (291.2 mg, 96.5%) by the same manner as described in Preparative Example 55 except that methyl-3-(7-methoxy-2-p-tolylbenzofuran-5-yl)propanoate prepared in Preparative Example 40 was used.

¹H NMR (CDCl₃, 400 MHz) δ 7.17 (1H, s, aromaticH), 7.03 (3H, d, *J* = 14.4 Hz, aromaticH), 6.58 (1H, s, aromaticH), 6.55 (1H, s, aromaticH), 5.57 (1H, s, OH), 3.87 (3H, s, CH₃), 3.67 (3H, s, CH₃), 2.86 (6H, s, CH₂), 2.57 (2H, s, CH₂), 2.33(3H, s, CH₃).

### <Preparative Example 57> methyl-3-(4-hydroxy-3-methoxy-5-(4-methylpenethyl)phenyl)propanoate

The target compound was obtained as a white solid (28.3 mg, 67%) by the same manner as described in Preparative Example 55 except that methyl-3-(7-methoxy-2-m-tolylbenzofuran-5-yl)propanoate prepared in Preparative Example 39 was used.

¹H NMR (CDCl₃, 400 MHz) δ 7.76 (4H, s, aromaticH), 6.58 (1H, s, aromaticH), 6.52 (1H, s, aromaticH), 5.56 (1H, s, OH), 3.87 (3H, s, CH₃), 3.67 (3H, s, CH₃), 2.86 (6H, s, CH₂), 2.56 (2H, s, CH₂), 2.32 (3H, s, CH₃).

### <Preparative Example 58> methyl-3-(4-hydroxy-3-methoxy-5-(3-methoxypenethyl)phenyl)propanoate

The target compound was obtained as a white solid (154.5 mg, 69.8%) by the same manner as described in Preparative Example 55 except that methyl-3-(7-methoxy-2-(3-methoxyphenyl)benzofuran-5-yl)propanoate prepared in Preparative Example 41 was used.

¹H NMR (CDCl₃, 400 MHz) δ 7.20 (1H, s, aromaticH), 6.83 (1H, s, aromaticH), 6.76 (2H, s, aromaticH), 6.58 (1H, s, aromaticH), 6.52 (1H, s, aromaticH), 5.57 (1H, s, OH), 3.87 (3H, s, CH₃), 3.79 (3H, s, CH₃), 3.67 (3H, s, CH₃), 2.85 (6H, d, *J* = 16 Hz, CH₂), 2.56 (2H, s, CH₂).

### <Preparative Example 59> 2-(3,4-dimethoxyphenyl)-7-methoxy-5-vinylbenzofuran

2-(3,4-dimethoxyphenyl)-7-methoxybenzofuran-5-carbaldehyde (60 mg, 0.19 mmol) prepared in Preparative Example 1 and methyltriphenylphosphonium iodine (116 mg, 0.29 mmol) were dissolved in dimethylformamide (5 mL), to which sodium hydride (0.023 g, 0.95 mmol) was added at 0°C, followed by stirring at room temperature for 12 hours. The reaction mixture was extracted with ethyl acetate and brine. The organic layer was separated and washed with water, dried over anhydrous magnesium sulfate, filtered, and concentrated under reduced pressure. The residue was purified by silica gel column chromatography (hexane:ethylacetate = 7:3) to give the target compound as a white solid (0.05 mg, 83.3%).

¹H NMR (CD₃OD, 500 MHz) δ 7.47 (1H, dd, J = 2.0 Hz, 8.0 Hz, aromatic-H), 7.44 (1H, d, J = 2.0 Hz, aromatic-H), 7.18 (1H, s, aromatic-H), 7.04 (1H, d, J = 8.5 Hz, aromatic-H), 7.02 (1H, s, aromatic-H), 6.98 (1H, s, aromatic-H), 6.79 (1H, dd, J = 10.5 Hz, 17.5 Hz, -CH=), 5.74 (1H, d, J = 17.5 Hz, trans-CH=), 5.18 (1H, d, J = 11.0 Hz, cis-CH=), 4.04 (3H, s, CH3), 3.93 (3H, s, CH3), 3.88 (3H, s, CH3);

### <Preparative Example 60> 7-methoxy-2-(4-methoxy-2-methylphenyl)-5-vinylbenzofuran

The target compound was obtained (0.055 g, 91.6%) by the same manner as described in Preparative Example 44 except that 7-methoxy-2-(4-methoxy-2-methylphenyl)benzofuran-5-carbaldehyde (60 mg, 0.20 mmol) prepared in Preparative Example 2 was used instead of 2-(3,4-dimethoxyphenyl)-7-methoxybenzofuran-5-carbaldehyde.

¹H NMR (CD₃OD, 400 MHz) δ 7.79 (1H, d, J = 8 Hz, aromaticH), 7.20 (1H, d, J = 1.2 Hz, aromaticH), 6.90 (1H, d, J = 1.2 Hz, aromaticH), 6.746.90 (4H, m, aromaticH,=CH), 5.71 (1H, d, J = 17.6 Hz, =CH₂), 5.22 (1H, d, J =11.2 Hz, =CH₂), 4.06 (3H, s, CH₃), 3.84 (3H, s, CH₃), 3.88 (3H, s, CH₃), 2.54 (3H, s, CH₃);

### <Preparative Examples 61 ∼ 65>

The compounds shown in Table 4 were prepared by the same manner as described in Example 1.

**[Table 4]**

| Prepara tive Example | Compound | Yield | ¹H NMR |
|---|---|---|---|
| 61 | 3-(2-(4-(tert-butyldimethylsi loxy)-3-methoxyphenyl)-7-methoxybenzofur an-5-yl)propane-1-ol | 89.1% | δ 7.32-7.35 (2H, m, aromatic-H), 6.98 (1H, d, *J* = 0.8 Hz, aromatic-H), 6.89 (1H, d, *J* = 3.2 Hz, aromatic-H), 6.81 (1H, s, aromatic-H), 6.63 (1H, s, aromatic-H), 4.04 (3H, s, CH₃), 3.90 (3H, s, CH₃), 3.72 (2H, q, *J* = 12.0 Hz, CH₂), 2.78 (2H, t, *J* = 3.6 Hz, CH₂), 1.92-1.99 (2H, m, CH₂), 1.01 (9H, d, *J* = 2.4 Hz, CH₃), 0.19 (6H, t, *J* = 2.4 Hz, CH₃); |
| 62 | 3-(2-(3-(tert-butyldimethylsi loxy)-4-methoxyphenyl)-7-methoxybenzofur an-5-yl)propane-1-ol | 87.1% | δ 7.46 (1H, dd, *J*₁ = 2.0 Hz, *J*₂ = 2.4 Hz, aromatic-H), 7.32 (1H, d, *J* = 2.4 Hz, aromatic-H), 6.96 (1H, d, *J* = 0.8 Hz, aromatic-H), 6.89 (1H, d, *J* = 8.4 Hz, aromatic-H), 6.77 (1H, s, aromatic-H), 6.62 (1H, d, *J* = 1.2 Hz, aromatic-H), 4.04 (3H, s, CH₃), 3.84 (3H, s, CH₃), 3.71 (2H, q, *J* = 12.0 Hz, CH₂), 2.77 (2H, t, *J* = 7.6 Hz, CH₂), 1.91-1.98 (2H, m, CH₂), 1.02 (9H, d, *J* = 2.8 Hz, CH₃), 0.19 (6H, t, *J* = 2.4 Hz, CH₃); |
| 63 | 3-(2-(3,5-bis(tert-butyldimethylsi lyloxy)phenyl)-7-methoxybenzofur an-5-yl)propane-1-ol | 94.7% | δ 6.98 (1H, d, *J* = 1.2 Hz, aromatic-H), 6.97 (1H, d, *J* = 2.4 Hz, aromatic-H), 6.87 (1H, s, aromatic-H), 6.65 (1H, d, *J* = 1.6 Hz, aromatic-H), 6.32 (1H, t, *J* = 2.0 Hz, aromatic-H), 4.04 (3H, s, CH₃), 3.71 (2H, d, *J* = 4.0 Hz, CH₂), 2.78 (2H, t, *J* = 3.6 Hz, CH₂), 1.91-1.98 (2H, m, CH₂), 1.00 (18H, t, *J* = 5.4 Hz, CH₃), 0.23 (12H, t, *J* = 3.2 Hz, CH₃); |
| 64 | 3-(2-(3,4-bis(tert-butyldimethylsi lyloxy)phenyl)-7-methoxybenzofur an-5-yl)propane-1-ol | 81.9% | δ 7.035 (1H, dd, *J* = 2.0 Hz, *J* = 2.0 Hz, aromatic-H), 7.29 (1H, d, *J* = 2.4 Hz, aromatic-H), 6.96 (1H, s, aromatic-H), 6.87 (1H, d, *J* = 8.4 Hz, aromatic-H), 6.75 (1H, s, aromatic-H), 6.62 (1H, d, *J* = 1.2 Hz, aromatic-H), 4.04 (3H, s, CH₃), 3.71 (2H, d, *J* = 4.0 Hz, CH₂), 2.77 (2H, t, *J* = 3.6 Hz, CH₂), 1.91-1.98 (2H, m, CH₂), 0.97-1.02 (18H, m, CH₃), 0.21-0.25 (12H, m, CH₃); |
| 65 | 3-(2-(4-(tert-butyldimethylsi lyloxy)-3,5-dimethoxyphenyl )-7-methoxybenzofur an-5-yl)propane-1-ol | 82.4% | δ 7.04 (2H, s, aromatic-H), 6.98 (1H, d, *J* = 1.2 Hz, aromatic-H), 6.83 (1H, s, aromatic-H), 6.34 (1H, d, *J* = 1.6 Hz, aromatic-H), 4.04 (3H, s, CH₃), 3.88 (6H, s, CH₃), 3.72 (2H, t, *J* = 2.4 Hz, CH₂), 2.78 (2H, t, *J* = 8.0 Hz, CH₂), 1.93-1.99 (2H, m, CH₂), 1.02 (9H, t, *J* = 3.2 Hz, CH₃), 0.15 (9H, t, *J* = 3.2 Hz, CH₃); |

### <Preparative Example 66> 2-(3,4-dimethoxyphenyl)-7-hydroxybenzofuran-5-carbaldehyde

3,4-dihydroxy-5-iodobenzaldehyde (250 mg, 0.96 mmol), 10% palladium/carbon (34 mg, 0.03 mmol), triphenylphosphine (34 mg, 0.13 mmol), copper iodide (12 mg, 0.06 mmol), triethylamine (329 mg, 3.0 mmol), and water were added in a 50 ml sealed tube containing a magnetic stirrer, followed by stirring for 1 hour with degassing using argon. 4-ethinyl-1,2-dimethoxybenzene (437 mg, 2.70 mmol) was added to the reaction mixture, followed by degassing with argon gas for 15 minutes. Then, the mixture was stirred at a high temperature for 24 hours. Upon completion of the reaction, the reaction mixture was extracted with ethyl acetate and the extract was washed with brine. The organic layer was separated and washed with water, dried over anhydrous magnesium sulfate, filtered, and concentrated under reduced pressure. The residue was purified by silica gel column chromatography (hexane:ethylacetate = 5:5) to give the target compound as a yellow solid (120 mg, 40%).

¹H NMR (CD₃OD, 400 MHz) δ 9.91 (1H, s, CHO), 7.66 (1H, d, *J* = 1.2 Hz, aromatic-H), 7.29-7.55 (1H, m, aromatic-H), 7.25 (1H, d, *J* = 0.8 Hz, aromatic-H), 7.18 (1H, s, aromatic-H), 7.07 (1H, d, *J* = 7.6 Hz aromatic-H), 3.94 (3H, s, CH₃), 3.89 (3H, s, CH₃);

### <Preparative Example 67> (E)-methyl-3-(2-(3,4-dimethoxyphenyl)-7-hydroxybenzofuran-5-yl)acrylate

2-(3,4-dimethoxyphenyl)-7-hydroxybenzofuran-5-carbaldehyde (110 mg, 0.37 mmol) prepared in Preparative Example 66 was dissolved in dichloromethane (5 mL), to which methyl(triphenylphosphoranyliden)acetate (308 mg, 0.92 mmol) was added, followed by stirring at a high temperature for 12 hours. Upon completion of the reaction, the reaction mixture was concentrated under reduced pressure. The residue was purified by silica gel column chromatography (hexane:ethylacetate = 7:3) to give the target compound as a white solid (70 mg, 54%).

¹H NMR (CD₃OD, 400 MHz) δ 7.65 (1H, d, *J* = 15.6 Hz, =CH), 7.45-7.48 (2H, m, aromatic-H), 7.38 (1H, s, aromatic-H), 7.29 (1H, s, aromatic-H), 7.07 (1H, d, *J* = 8.0 Hz, aromatic-H), 7.01 (1H, d, *J* = 1.2 Hz, aromatic-H), 6.44 (1H, d, *J* = 16.0 Hz, =CH), 3.85 (3H, s, CH₃), 3.80 (3H, s, CH₃), 3.71 (3H, s, CH₃);

### <Preparative Example 68> methyl-3-(2-(3,4-dimethoxyphenyl)-7-hydroxybenzofuran-5-yl)propanoate

(E)-methyl-3-(2-(3,4-dimethoxyphenyl)-7-hydroxybenzofuran-5-yl)acrylate (60 mg, 0.17 mmol) prepared in Preparative Example 67 was dissolved in tetrahydrofuran (5 mL), to which palladium/carbon (29 mg, 0.014 mmol, 10 wt%) was added with a couple of drops of acetic acid, followed by stirring at room temperature for 30 minutes in the presence of hydrogen. Upon completion of the reaction, the reaction mixture was concentrated under reduced pressure. The residue was purified by silica gel column chromatography (hexane:ethylacetate = 7:3) to give the target compound as a white solid (56 mg, 93.3%) .

¹H NMR (CD₃OD, 400 MHz) δ 9.99 (1H, s, aromatic-OH), 7.43 (1H, dd, *J*₁ = 1.6 Hz, *J*₂ = 2.0 Hz, aromatic-H), 7.41 (1H, m, d, *J* = 2.0 Hz, aromatic-H), 7.17 (1H, s, aromatic-H), 7.06 (1H, d, *J* = 8.4 Hz, aromatic-H), 6.84 (1H, d, *J* = 0.8 Hz, aromatic-H), 6.56 (1H, d, *J* = 1.2 Hz, aromatic-H), 3.85 (3H, s, CH₃), 3.79 (3H, s, CH₃), 3.57 (3H, s, CH₃), 2.82 (2H, t, *J* = 3.6 Hz, CH₂), 2.61 (2H, t, *J* = 3.6 Hz, CH₂);

### <Example 1> 3-[2-(3,4-dimethoxyphenyl)-7-methoxybenzofuran-5-yl]propane-1-ol

Methyl 3-(2-(3,4-dimethoxyphenyl)-7-methoxybenzofuran-5-yl]propanoate (57 mg, 0.15 mmol) prepared in Preparative Example 27 was dissolved in tetrahydrofuran (5 mL), to which lithium aluminum hydride (2.0 M, 0.15 mL, 0.31 mmol) dissolved in tetrahydrofuran was slowed added at 0°C, followed by stirring until the completion of the reaction was confirmed with thin layer chromatography. Upon completion of the reaction, 10% HCl was added to the mixture, and ethyl acetate and brine were also added thereto. The organic layer was separated and washed with water, dried over anhydrous magnesium sulfate, filtered, and concentrated under reduced pressure. The residue was purified by silica gel column chromatography (hexane:ethylacetate = 5:5) to give the target compound as a white solid (46 mg, 87.5%).

¹H NMR (CDCl₃, 400 MHz) δ 7.45 (1H, dd, *J* = 2.0 Hz, 8.5 Hz, aromaticH), 7.37 (1H, d, *J* = 2.0 Hz, aromaticH), 6.98 (1H, d, *J* = 1.2 Hz aromaticH), 6.93 (1H, d, *J* = 8.4 Hz, aromaticH), 6.84 (1H, s, aromaticH), 6.64 (1H, d, *J* = 1.2 Hz, aromaticH), 4.04 (3H, s, CH₃), 3.99 (3H, s, CH₃), 3.93 (3H, s, CH₃), 3.72 (2H, q, *J* = 5.6 Hz, CH₂), 2.79 (2H, t, *J* = 8 Hz, CH₂), 1.921.99 (2H, m, CH₂);

### <Examples 2 ∼ 13>

The compounds shown in Table 5 were prepared by the same manner as described in Example 1.

**[Table 5]**

| Example | Compound | Yield | ¹H NMR |
|---|---|---|---|
| 2 | 3-(7-methoxy-2-(4-methoxy-2-methylphenyl)b enzofuran-5-yl)propane-1-ol | 89.1% | δ7.78 (1H, d, *J* = 2.0 Hz, 7.2 Hz, aromaticH), 7.01 (1H, d, *J* = 1.2 Hz, aromaticH), 6.826.84 (2H, m, aromaticH), 6.72 (1H, s, aromaticH), 6.65 (1H, d, *J* = 1.2 Hz, aromaticH), 4.04 (3H, s, CH₃), 3.84 (3H, s, CH₃), 3.72 (2H, d, *J* = 5.2 Hz, 6.0 Hz, CH₂), 2.81 (2H, t, *J* = 7.6 Hz, CH₂), 1.922.04 (2H, m, CH₂); |
| 3 | 3-(7-methoxy-2-m-tolylbenzofura n-5-yl)propane-1-ol | 50% | δ7.71 (1H, s, aromaticH) 7.66 (1H, d, *J* = 8 Hz, aromaticH), 7.31, (1H, t, *J* = 8 Hz, aromaticH), 7.15 (1H, d, *J* = 8 Hz , aromaticH), 7.00 (1H, s, aromaticH), 6.93 (1H, s, aromaticH), 6.65 (1H, s, aromaticH), 4.05 (3H, s, CH₃), 3.71 (2H, q, *J* = 8 Hz, CH₂), 2.79 (2H, t, *J* = 8 Hz, CH₂), 2.41 (3H, s, CH₃), 1.931.97 (2H, m, CH₂), 0.88 (1H, t, *J* = 8 Hz, OH) |
| 4 | 3-(7-methoxy-2-p-tolylbenzofura n-5-yl)propane-1-ol | 80.3% | δ7.76 (2H, d, *J* = 8.0 Hz, aromaticH), 7.23 (2H, d, *J* = 8 Hz, aromaticH), 6.99 (1H, s, aromaticH), 6.89 (1H, s, aromaticH), 6.64 (1H, s, aromaticH), 4.04 (3H, s, CH₃), 3.71 (2H, q, *J* = 8 Hz, CH₂), 2.78 (2H, t, *J* = 8 Hz, CH₂), 2.39 (3H, s, CH₃), 1.911.98 (2H, m, CH₂), 1.27 (1H, t, *J* = 8 Hz, OH), |
| 5 | 3-(7-methoxy-2-(3-methoxyphenyl) benzofuran-5-yl)propane-1-ol | 75.8% | δ7.46 (1H, d, *J* = 8 Hz, aromaticH), 7.40 (1H, s, aromaticH), 7.34 (1H, t, *J* = 8 Hz, aromaticH), 7.00 (1H, s, aromaticH), 6.95 (1H, s, aromaticH) 6.89 (1H, q, *J* = 8 Hz, aromaticH), 6.66 (1H, s, aromaticH), 4.04 (3H, s, CH₃), 3.89 (3H, s, CH₃),). 3.71 (2H, q, *J* = 8 Hz, CH₂), 2.79 (2H, t, *J* = 8 Hz, CH₂), 1.931.99 (2H, m, CH₂), 1.27 (1H, t, *J* = 8 Hz, OH), |
| 6 | 3-[2-(3,5-difluorophenyl )-7-methoxybenzofu ran-5-yl]propane-1-ol | 28.5% | δ7.39 (1H, d, *J* = 2.0 Hz, aromaticH), 7.36 (1H, d, *J* = 2.0 Hz, aromaticH), 7.02 (1H, s, aromaticH), 6.98 (1H, s, aromaticH), 6.77 (1H, t, *J* = 2.0 Hz, 6.8 Hz, aromaticH), 6.70 (1H, s, aromaticH), 4.04 (3H, s, CH₃), 3.72 (2H, q, *J* = 6.0 Hz, CH₂), 2.79 (2H, t, *J* = 7.5 Hz, CH₂), 1.882.04 (2H, m, CH₂); |
| 7 | 3-(7-methoxy-2-(4-methoxyphenyl) benzofuran-5-yl)propane-1-ol | 73.8% | δ7.79 (2H, t, J = 8.0 Hz, aromaticH), 6.99 (2H, d, *J* = 4 Hz, aromaticH), 6.96 (2H, d, *J* = 4 Hz, aromaticH), 6.84 (1H, s, aromaticH), 6.63 (1H, s, aromaticH), 4.04 (3H, s, CH₃), 3.85 (3H, s, CH₃), 3.71 (2H, q, *J* = 8 Hz, CH₂), 2.78 (2H, t, *J* = 8 Hz, CH₂), 1.911.98 (2H, m, CH₂), 1.26 (1H, t, *J* = 8 Hz, OH); |
| 8 | 3-(2-(3-fluorophenyl)-7-methoxybenzofu ran-5-yl)propane-1-ol | 95.8% | δ7.64 (1H, d, *J* = 8 Hz, aromaticH) 7.57 (1H, d, *J* = 8 Hz, aromaticH), 7.39, (1H, q, *J* = 8 Hz, aromaticH), 7.017.05 (2H, m, aromaticH), 6.97 (1H, s, aromaticH), 4.04 (3H, s, CH₃), 3.72 (2H, q, *J* = 8 Hz, CH₂), 2.79 (2H, t, *J* = 8 Hz, CH₂), 1.911.99 (2H, m, CH₂), 1.27 (1H, q, *J* = 8 Hz, OH); |
| 9 | 3-(2-(4-(dimethylamino )phenyl)-7-methoxybenzofu ran-5-yl)propane-1-ol | 78.6% | δ7.74 (2H, d, *J* = 8.0 Hz, aromaticH), 6.95 (2H, s, aromaticH), 6.75 (2H, d, *J* = 8 Hz, aromaticH), 6.72 (1H, s, aromaticH), 6.59 (1H, s, aromaticH), 4.04 (3H, s, CH₃), 3.71 (2H, q, *J* = 8 Hz, CH₂), 3.01 (6H, s, CH₃), 2.77 (2H, t, *J* = 8 Hz, CH₂), 1.911.98 (2H, m, CH₂), 1.265(1H, t, *J* = 8 Hz, OH); |
| 10 | 3-(7-methoxy-2-(2-methoxyphenyl) benzofuran-5-yl)propane-1-ol | 85% | δ8.10 (1H, dd, *J* = 1.6, 1.6 Hz aromaticH), 7.297.31 (1H, m, aromaticH), 7.25 (1H, d, *J* = 0.8 Hz, aromaticH), 7.06 (1H, t, *J* = 8 Hz, aromaticH), 7.01 (2H, dd, *J* = 0.8, 8.4 Hz aromaticH), 6.64 (1H, s, aromaticH), 4.04 (3H, s, CH₃), 3.99 (3H, s, CH₃), 3.71 (2H, q, *J* = 8 Hz, CH₂), 2.78 (2H, t, *J* = 8 Hz, CH₂), 1.911.97 (2H, m, CH₂), 0.885 (1H, t, *J* = 8 Hz, OH); |
| 11 | 3-(7-methoxy-2-(3-(trifluorometh yl)phenyl)benz ofuran-5-yl)propane-1-ol | 79.4% | δ8.10 (1H, s, aromaticH) 8.03 (1H, d, *J* = 7.2 Hz, aromaticH), 7.527.59 (2H, m, aromaticH), 7.03 (2H, d, *J* = 6.4 Hz, aromaticH), 6.69 (1H, d, *J* = 1.2 Hz, aromaticH), 4.05 (3H, s, CH₃), 3.72 (2H, d, *J* = 2.4 Hz, CH₂), 2.80 (2H, t, *J* = 8 Hz, CH₂), 1.921.99 (2H, m, CH₂), 1.28(1H, d, *J* = 29.2 Hz, OH); |
| 12 | 3-[2-(3,5-dimethoxypheny l)-7-methoxybenzofu ran-5-yl]propane-1-ol | 90.1% | δ7.02 (1H, d, *J* = 2.0 Hz, aromaticH), 7.00 (1H, d, *J* = 1.2 Hz, aromaticH), 6.93 (1H, s, aromaticH), 6.66 (1H, d, *J* = 0.8 Hz, aromaticH), 6.46 (1H, t, *J* = 3.0 Hz, aromaticH), 4.04 (3H, s, CH₃), 3.86 (6H, s, CH₃), 3.72 (2H, q, *J* = 5.2 Hz, CH₂), 2.78 (2H, t, *J* = 8 Hz, CH₂), 1.911.98 (2H, m, CH₂), 1.31 (1H, t, *J* = 4.8 Hz, OH) |
| 13 | 3-(7-methoxy-2-(4-(trifluorometh yl)phenyl)benz ofuran-5-yl)propane-1-ol | 79.4% | δ7.97 (2H, d, *J* = 8.8 Hz, aromaticH) 7.67 (2H, d, *J* = 8.4 Hz, aromaticH), 7..06 (1H, s, aromaticH), 7.03 (1H, s, aromaticH), 6.69 (1H, d, *J* = 1.2 Hz, aromaticH), 4.04 (3H, s, CH₃), 3.72 (2H, d, *J* = 6.4 Hz, CH₂), 2.80 (2H, t, *J* = 8 Hz, CH₂), 1.921.99 (2H, m, CH₂), 1.35(1H, s, OH) ; |

### <Example 14> 2-(3,4-dimethoxyphenethyl)-4-(3-hydroxypropyl)-6-methoxyphenol

Methyl-3-(3-(3,4-dimethoxyphenethyl)-4-hydroxy-5-methoxyphenyl)propanoate (50 mg) prepared in Preparative Example 55 was dissolved in tetrahydrofuran (3 mL), to which lithium aluminum hydride (2.0 M, 1.0 equivalent) dissolved in tetrahydrofuran was slowed added at 0°C, followed by stirring until the completion of the reaction was confirmed with thin layer chromatography. Upon completion of the reaction, 10% HCl was added to the mixture. The reaction mixture was extracted with ethyl acetate and brine. The organic layer was separated and washed with water, dried over anhydrous magnesium sulfate, filtered, and concentrated under reduced pressure. The residue was purified by silica gel column chromatography to give the target compound as a white solid (38 mg, 83.5%).

¹H NMR (CDCl₃, 500 MHz) δ 7.45 (1H, dd, *J* = 2.0 Hz, 8.5 Hz, aromaticH), 7.37 (1H, d, *J* = 2.0 Hz, aromaticH), 6.98 (1H, s, aromaticH), 6.93 (1H, d, *J* = 8.5 Hz, aromaticH), 6.84 (1H, s, aromaticH), 6.64 (1H, d, *J* = 0.5 Hz, aromaticH), 4.04 (3H, s, CH₃), 3.99 (3H, s, CH₃), 3.93 (3H, s, CH₃), 3.72 (2H, t, *J* = 6.5 Hz, CH₂), 2.79 (2H, t, *J* = 7.5 Hz, CH₂), 1.931.98 (2H, m, CH₂);

### <Example 15> 4-(3-hydroxypropyl)-2-methoxy-6-(3-methylpenethyl)phenol

The target compound was obtained (246 mg, 92.5%) by the same manner as described in Example 14 except that methyl-3-(4-hydroxy-3-methoxy-5-(3-methylpenethyl)phenyl)propanoate prepared in Preparative Example 56 was used instead of methyl-3-(3-(3,4-dimethoxyphenethyl)-4-hydroxy-5-methoxyphenyl)propanoate.

¹H NMR (CDCl₃, 400 MHz) δ 7.17 (1H, *J* = 8Hz, aromaticH) 7.056.99 (3H, m, aromaticH), 6.58 (1H, s, aromaticH), 6.53 (1H, s, aromaticH), 5.55 (1H, s, OH), 3.87 (3H, s, CH₃), 3.693.62 (2H, m, CH₂), 2.87 (4H, q , *J* = 12 Hz, CH₂). 2.60 (2H, t, *J* = 8 Hz, CH₂) 2.33 (3H, s, CH₃), 1.871.80 (2H, m, CH₂), 0.88 (1H, t, *J* = 8Hz, OH).

### <Example 16> 4-(3-hydroxypropyl)-2-methoxy-6-(4-methylpenethyl)phenol

The target compound was obtained as a white solid (79.7 mg, 67.3%) by the same manner as described in Example 14 except that methyl-3-(4-hydroxy-3-methoxy-5-(4-methylpenethyl)phenyl)propanoate prepared in Preparative Example 57 was used instead of methyl-3-(3-(3,4-dimethoxyphenethyl)-4-hydroxy-5-methoxyphenyl)propanoate.

¹H NMR (CDCl₃, 400 MHz) δ 7.12-7.07 (4H, m, aromaticH), 6.57 (1H, s, aromaticH), 6.52 (1H, s, aromaticH), 5.54 (1H, s, OH), 3.87 (3H, s, CH₃), 3.63 (2H, q, *J* = 18 Hz, CH₂), 2.872 (4H, s , CH₂), 2.59 (2H, t, *J* = 8 Hz, CH₂), 2.31 (3H, s, CH₃), 1.86-1.80 (2H, m, CH₂), 1.18 (1H, t, *J* = 8Hz, OH).

### <Example 17> 4-(3-hydroxypropyl)-2-methoxy-6-(3-methoxypenethyl)phenol

The target compound was obtained as a white solid (130 mg, 92.5%) by the same manner as described in Example 14 except that methyl-3-(4-hydroxy-3-methoxy-5-(3-methoxypenethyl)phenyl)propanoate prepared in Preparative Example 58 was used instead of methyl-3-(3-(3,4-dimethoxyphenethyl)-4-hydroxy-5-methoxyphenyl)propanoate.

¹H NMR (CDCl₃, 400 MHz) δ 7.19 (1H, t, *J* = 8Hz, aromaticH), 6.82 (1H, d, *J* = 8Hz, aromaticH), 6.74 (2H, d, *J* = 12Hz, aromaticH), 6.58 (1H, s, aromaticH), 6.51 (1H, s, aromaticH), 5.55 (1H, s, OH), 3.87 (3H, s, CH₃), 3.78 (3H, s, CH₃), 3.65 (2H,q, *J* = 16 Hz, CH₂), 2.89 (4H, s , CH₂), 2.59 (2H, t, *J* = 8 Hz, CH₂), 1.861.79 (2H, m, CH₂), 1.23 (1H, t, *J* = 10Hz, OH).

### <Example 18> [2-(3,4-dimethoxyphenyl)-7-methoxybenzofuran-5-yl]methanol

2-(3,4-dimethoxyphenyl)-7-methoxybenzofuran-5-carbaldehyde (0.1 g, 0.32 mmol) prepared in Preparative Example 1 was dissolved in tetrahydrofuran (5 mL), to which sodium borohydride (0.036 g, 0.96 mmol) was added at 0°C, followed by stirring until the completion of the reaction was confirmed with thin layer chromatography. Upon completion of the reaction, 10% HCl was added to the mixture. The reaction mixture was extracted with ethyl acetate and brine. The organic layer was separated and washed with water, dried over anhydrous magnesium sulfate, filtered, and concentrated under reduced pressure. The residue was purified by silica gel column chromatography (hexane:ethylacetate = 6:4) to give the target compound as a white solid (85 mg, 84.6%).

¹H-NMR (CDCl₃, 500 MHz) δ 7.50 (1H, dd, *J* = 2.0 Hz, 8.0 Hz, aromatic-H), 7.47 (1H, d, *J* = 1.5 Hz, aromatic-H), 7.15 (1H, s, aromatic-H), 7.12 (1H, s, aromatic-H), 7.07 (1H, d, *J* = 8.5 Hz, aromatic-H), 6.92 (1H, s, aromatic-H), 4.68 (2H, d, *J* = 6.0 Hz, CH₂), 4.14 (1H, t, *J* = 6.0 Hz, OH), 4.02 (3H, s, CH₃), 3.92 (3H, s, CH₃), 3.87 (3H, s, CH₃) ;

### <Example 19> (7-methoxy-2-(4-methoxy-2-methylphenyl)benzofuran-5-yl)methanol

The target compound was obtained as a white solid by the same manner as described in Example 18 except that 7-methoxy-2-(4-methoxy-2-methylphenyl)benzofuran-5-carbaldehyde (0.1 g, 0.35 mmol) prepared in Preparative Example 2 was used instead of 2-(3,4-dimethoxyphenyl)-7-methoxybenzofuran-5-carbaldehyde (0.1 g, 0.32 mmol).

¹H-NMR (CDCl₃, 400 MHz) δ 7.80 (1H, d, *J* = 8.0 Hz, aromatic-H), 7.17 (1H, s, aromatic-H), 6.83 (13H, d, *J =* 8.8 Hz, aromatic-H), 6.75 (1H, s, aromatic-H), 4.76 (2H, d, *J* = 6.0 Hz, CH₂), 4.05 (3H, s, CH₃), 3.85 (3H, s, CH₃), 2.55 (3H, s, CH₃), 2.64 (1H, t, *J* = 12.4 Hz, OH),;

### <Example 20> 2-[2-(3,4-dimethoxyphenyl)-7-methoxybenzofuran-5-yl]ethanol

2-(3,4-dimethoxyphenyl)-7-methoxy-5-vinylbenzofuran (30 mg, 0.10 mmol) prepared in Preparative Example 59 was dissolved in tetrahydrofuran (2 mL), to which borane tetrahydrofuran complex (1.0 M, 0.11 mL, 0.11 mmol) dissolved in tetrahydrofuran was added at 0°C, followed by stirring at room temperature for 3 hours. Then, 0.1 ml of 10% NaOH solution and 0.1 ml of 30% H₂O₂ were added thereto, followed by stirring at room temperature for 30 minutes and stirring at 55°C for 1 hour. Upon completion of the reaction, the reaction mixture was extracted with ethyl acetate and brine. The organic layer was separated and washed with water, dried over anhydrous magnesium sulfate, filtered, and concentrated under reduced pressure. The residue was purified by silica gel column chromatography (hexane:ethylacetate = 5:5) to give the target compound as a white solid (0.014 g, 43.8%).

¹H NMR (CD₃OD, 500 MHz) δ 7.46 (1H, dd, *J* = 2.0 Hz, 8.5 Hz, aromaticH), 7.44 (1H, d, *J* = 2.0 Hz, aromaticH), 7.03 (1H, d, *J* = 8.0 Hz, aromaticH), 7.01 (1H, s, aromaticH), 6.98 (1H, s, aromaticH), 6.74 (1H, d, *J* = 0.5 Hz, aromaticH), 4.01 (3H, s, CH₃), 3.92 (3H, s, CH₃), 3.87 (3H, s, CH₃), 3.79 (2H, t, *J* = 7.0 Hz, CH₂OH), 2.88 (2H, t, *J* = 7.0 Hz, CH₂) ;

### <Example 21> 2-(7-methoxy-2-(4-methoxy-2-methylphenyl)benzofuran-5-yl]ethanol

The target compound was obtained as a white solid (0.016 g, 50.1%) by the same manner as described in Example 20 except that 7-methoxy-2-(4-methoxy-2-methylphenyl)-5-vinylbenzofuran prepared in Preparative Example 60 was used instead of 2-(3,4-dimethoxyphenyl)-7-methoxy-5-vinylbenzofuran.

¹H NMR (CD₃OD, 400 MHz) δ 7.78 (1H, d, *J* = 9.6 Hz, aromaticH), 7.04 (1H, s, aromaticH), 6.83 (2H, d, *J* = 7.6 Hz, aromaticH), 6.71 (1H, s, aromaticH), 6.66 (1H, d, *J* = 1.2 Hz aromaticH), 4.04 (3H, s, CH₃), 3.91 (2H, s, CH₂), 3.84 (3H, s, CH₃), 2.95 (2H, t, *J* = 6.4 Hz, CH₂), 2.54 (3H, s, CH₃) ;

### <Example 22> 4-(5-(3-hydroxypropyl)-7-methoxybenzofuran-2-yl)-2-methoxyphenol

3-(2-(4-(tert-butyldimethylsiloxy)-3-methoxyphenyl)-7-methoxybenzofuran-5-yl)propane-1-ol (70 mg, 0.16 mmol) prepared in Preparative Example 61 was dissolved in tetrahydrofuran (5 mL), to which tetrabutylammonium fluoride (0.32 mmol) was added at 0°C, followed by stirring at room temperature for 30 minutes. Upon completion of the reaction, the reaction mixture was concentrated under reduced pressure. The residue was purified by silica gel column chromatography (hexane:ethylacetate = 5:5) to give the target compound as a white solid (45 mg, 86.1%).

¹H NMR (CD₃OD, 400 MHz) δ 7.39 (1H, dd, J₁ = 2.0 Hz, J₂ = 2.0 Hz, aromatic-H), 7.37 (1H, d, J = 1.2 Hz, aromatic-H), 6.97 (2H, d, J = 7.6 Hz, aromatic-H), 6.81 (1H, s, aromatic-H), 6.63 (1H, s, aromatic-H), 4.03 (3H, s, CH3), 3.99 (3H, s, CH3), 3.71 (2H, t, J = 6.4 Hz, CH2), 2.78 (2H, t, J = 3.6 Hz, CH2), 1.91-1.98 (2H, m, CH2);

### <Example 23> 5-(5-(3-hydroxypropyl)-7-methoxybenzofuran-2-yl)-2-methoxyphenol

The target compound was obtained (40 mg, 67.8%) by the same manner as described in Example 22 except that 3-(2-(3-(tert-butyldimethylsiloxy)-3-methoxyphenyl)-7-methoxybenzofuran-5-yl)propane-1-ol prepared in Preparative Example 62 was used instead of 3-(2-(4-(tert-butyldimethylsiloxy)-3-methoxyphenyl)-7-methoxybenzofuran-5-yl)propane-1-ol.

¹H NMR (CD₃OD, 400 MHz) δ 7.35 (1H, dd, *J*₁ = 2.4 Hz, *J*₂ = 1.6 Hz, aromatic-H), 7.30 (1H, d, *J* = 2.0 Hz, aromatic-H), 7.00 (1H, d, *J* = 8.4 Hz, aromatic-H), 6.97 (1H, d, *J =* 0.8 Hz, aromatic-H), 6.88 (1H, s, aromatic-H), 6.70 (1H, s, aromatic-H), 4.01 (3H, s, CH₃), 3.90 (3H, s, CH₃), 3.59 (2H, t, *J* = 7.6 Hz, CH₂), 2.74 (2H, t, *J* = 8.0 Hz, CH₂), 1.86-1.90 (2H, m, CH₂) ;

### <Example 24> 5-(5-(3-hydroxypropyl)-7-methoxybenzofuran-2-yl)benzene-1,3-diol

The target compound was obtained (90 mg, 85.5%) by the same manner as described in Example 22 except that 3-(2-(3,5-bis(tert-butyldimethylsilyloxy)phenyl)-7-methoxybenzofuran-5-yl)propane-1-ol prepared in Preparative Example 63 was used instead of 3-(2-(4-(tert-butyldimethylsiloxy)-3-methoxyphenyl)-7-methoxybenzofuran-5-yl)propane-1-ol.

¹H NMR (CD₃OD, 400 MHz) δ 7.35 (2H, s, OH), 7.12 (1H, s, aromatic-H), 6.97 (1H, s, aromatic-H), 6.74 (1H, s, aromatic-H), 6.72 (1H, d, *J* = 2.4 Hz, aromatic-H), 6.22 (1H, t, *J* = 2.4 Hz, aromatic-H), 4.46 (1H, t, *J* = 4.8 Hz , OH), 3.93 (3H, s, CH₃), 3.42 (2H, q, *J* = 6.4 Hz, CH₂), 2.65 (2H, t, *J* = 8.0 Hz, CH₂), 1.71-1.78 (2H, m, CH₂) ;

### <Example 25> 4-(5-(3-hydroxypropyl)-7-methoxybenzofuran-2-yl)benzene-1,2-diol

The target compound was obtained (70 mg, 74.5%) by the same manner as described in Example 22 except that 3-(2-(3,4-bis(tert-butyldimethylsilyloxy)phenyl)-7-methoxybenzofuran-5-yl)propane-1-ol prepared in Preparative Example 64 was used instead of 3-(2-(4-(tert-butyldimethylsiloxy)-3-methoxyphenyl)-7-methoxybenzofuran-5-yl)propane-1-ol.

¹H NMR (CD₃OD, 400 MHz) δ 7.12 (1H, d, *J* = 2.0 Hz, aromatic-H), 7.16 (1H, dd, *J* = 2.0 Hz, *J* = 2.0 Hz, aromatic-H), 6.98 (1H, s, aromatic-H), 6.94 (1H, s, aromatic-H), 6.81 (1H, d, *J* = 8.4 Hz, aromatic-H), 6.71 (1H, s , aromatic-H), 3.94 (3H, s, CH₃), 3.43 (2H, t, *J =* 6.8 Hz, CH₂), 2.66 (2H, t, *J* = 8.0 Hz, CH₂), 1.71-1.78 (2H, m, CH₂);

### <Example 26> 4-(5-(3-hydroxypropyl)-7-methoxybenzofuran-2-yl)-2,6-dimethoxyphenol

The target compound was obtained (80 mg, 74.8%) by the same manner as described in Example 22 except that 3-(2-(4-(tert-butyldimethylsilyloxy)-3,5-dimethoxyphenyl)-7-methoxybenzofuran-5-yl)propane-1-ol prepared in Preparative Example 65 was used instead of 3-(2-(4-(tert-butyldimethylsiloxy)-3-methoxyphenyl)-7-methoxybenzofuran-5-yl)propane-1-ol.

¹H NMR (CD₃OD, 400 MHz) δ 8.79 (1H, s, OH), 7.21 (1H, s, aromatic-H), 7.10 (2H, s, aromatic-H), 6.96 (1H, s, aromatic-H), 6.74 (1H, d, *J* = 1.2 Hz, aromatic-H), 6.22 (1H, t, *J* = 2.4 Hz, aromatic-H), 4.47 (1H, s , OH), 4.03 (3H, s, CH₃), 3.86 (6H, s, CH₃), 3.44 (2H, t, *J* = 6.4 Hz, CH₂), 2.67 (2H, t, *J* = 8.0 Hz, CH₂), 1.73-1.80 (2H, m, CH₂) ;

### <Example 27> 2-(3,4-dimethoxyphenethyl)-5-(3-hydroxypropyl)-7-ol

Methyl-3-(2-(3,4-dimethoxyphenyl)-7-hydroxybenzofuran-5-yl)propanoate (56 mg, 0.16 mmol) prepared in Preparative Example 68 was dissolved in tetrahydrofuran (5 mL), to which lithium aluminum hydride (2.0 M, 0.15 mL, 0.31 mmol) dissolved in tetrahydrofuran was slowed added at 0°C, followed by stirring until the completion of the reaction was confirmed with thin layer chromatography. Upon completion of the reaction, 10% HCl was added to the mixture, and ethyl acetate and brine were also added thereto. The organic layer was separated and washed with water, dried over anhydrous magnesium sulfate, filtered, and concentrated under reduced pressure. The residue was purified by silica gel column chromatography (hexane:ethylacetate = 5:5) to give the target compound as a white solid (36 mg, 67.9%).

¹H NMR (CD₃OD, 400 MHz) δ 9.88 (1H, s, aromatic-OH), 7.43 (1H, dd, *J*₁ = 1.6 Hz, *J*₂ = 2.0 Hz, aromatic-H), 7.41 (1H, m, d, *J* = 2.0 Hz, aromatic-H), 7.16 (1H, s, aromatic-H), 7.06 (1H, d, *J* = 8.4 Hz, aromatic-H), 6.81 (1H, d, *J* = 0.8 Hz, aromatic-H), 6.55 (1H, d, *J* = 1.2 Hz, aromatic-H), 3.85 (3H, s, CH₃), 3.79 (3H, s, CH₃), 3.36-3.44 (2H, m, CH₂), 2.57 (2H, t, *J* = 8.4 Hz, CH₂), 1.67-1.74 (2H, m, CH₂) ;

### <Example 28> 3-(2-(3,4-dimethoxyphenyl)-7-methoxybenzofuran-5-yl)propyl acetate

3-[2-(3,4-dimethoxyphenyl)-7-methoxybenzofuran-5-yl]propane-1-ol(50 mg, 0.09 mmol) prepared in Example 1 was dissolved in dichloromethane (5 mL), to which triethylamine was added, followed by stirring for 10 minutes. The mixture was added with acetylchloride at 0°C, followed by stirring at room temperature for 12 hours. Then, the reaction mixture was concentrated under reduced pressure. The residue was purified by silica gel column chromatography (hexane:ethylacetate = 6:4) to give the target compound as a white solid (40 mg, 71%).

¹H NMR (CD₃OD, 400 MHz) δ 7.45 (1H, dd, *J* = 2.0 Hz, *J* = 2.0 Hz, aromatic-H), 7.36 (1H, d, *J* = 2.0 Hz, aromatic-H), 6.96 (1H, d, *J* = 1.2 Hz, aromatic-H), 6.93 (1H, d, *J* = 8.4 Hz, aromatic-H), 6.84 (1H, s, aromatic-H), 6.61 (1H, d, *J =* 1.2 Hz, aromatic-H), 4.13 (2H, d, *J* = 6.8 Hz, CH₂), 4.04 (3H, s, CH₃), 3.99 (3H, s, CH₃), 3.93 (3H, s, CH₃), 2.76 (2H, d, *J* = 7.6 Hz, CH₂), 2.07 (3H, s, CH₃), 1.97-2.04 (2H, m, CH₂),

### <Experimental Example 1> Evaluation of cell survival rate of macrophages

Raw 264.7 (ATCC TIB71) cells, the mouse derived macrophages', were inoculated in a 96-well plate containing DMEM (Dulbecco's modified Eagle medium; Welgene, Korea) supplemented with 10% FBS (Fetal Bovine Serum), 2 mM glutamine, penicillin (100 unit/Mℓ), and streptomycin (100 µg/Mℓ) at the density of 1×10⁴ cells/well. The cells were cultured in 5% CO₂ at 37°C for 4 hours to attach the cells onto the plate. Next, the cells were treated with each sample at different concentrations, followed by further culture for 24 hours. To investigate the cell survival rate, 5 mg/Mℓ of MTT (3-(4,5-dimethylthiazol-2-yl)-2,5-diphenyltetrazoliumbromide, Amresco, OH, USA) was distributed thereto (10 µℓ/well), followed by culture for 4 hours. Then, the supernatant was eliminated. Formazan reduced from MTT by mitochondria was dissolved in 100 µℓ of DMSO and then OD₅₇₀ was measured. 0.2% DMSO was used for the negative control. Considering OD of the negative control as 100%, the cell survival rate was calculated and presented in Table 6.

**[Table 6]**

| Example | Grades of cell survival rate (%, in 10 µM) |
|---|---|
| 1* | A |
| 2* | A |
| 3* | A |
| 4 | A |
| 5* | A |
| 6* | A |
| 7* | A |
| 8* | A |
| 9* | A |
| 14* | C |
| 15* | A |
| 16* | A |
| 17* | B |
| 18* | A |
| 20* | B |
| 22* | A |
| 23* | B |
| 24* | A |
| 25 | A |
| 26* | A |
| 27* | B |
| 28* | A |

| | |
|---|---|
| * Compounds not within the claims | |

(As shown in Table 6, the cell survival rate (%) at the sample concentration of 10 µM was measured and sorted by the following grades:
A: 90 < A ≤ 100;
B: 80 < B ≤ 90; and
C: 70 < C ≤ 80.)

As shown in Table 6, when the 2-phenylbenzofuran derivative of the present invention was treated at the concentration of 10 µM, the cell survival rate (%) was excellent, indicating that it has a low effect on macrophages, in other words it has low cytotoxicity to macrophages, so that it can be effectively used as a pharmaceutical composition for preventing or treating inflammatory disease.

### <Experimental Example 2> Effect of the compound on the inflammation induced macrophages

### 1. Inhibition of nitrogen oxide (NO) generation

Raw 264.7 (ATCC TIB71) cells were inoculated in a 96-well plate containing the same medium as used in Experimental Example 1 at the density of 1×10⁵ cells/well. The cells were cultured in 5% CO₂ at 37°C for 4 hours to attach the cells onto the plate. The cells were treated with each sample at different concentrations, followed by further culture for 1 hour. Inflammation induced macrophages (LPS) were treated thereto at the final concentration of 500 ng/Mℓ, followed by further culture for 24 hours. 100 µℓ of the supernatant was taken and transferred in a new plate, which was mixed with Griess reagent (a mixture of 1% sulfanilamide, 0.1% N-[1-naphthyl]-ethylenediamine dihydrochloride, and 5% phosphoric acid), followed by reaction for 10 minutes. Then, OD₅₄₀ was measured. NO generation was quantified by using the sodium nitrite standard curve prepared from sodium nitrates dissolved in the same medium at different concentrations. Considering the NO generation of the inflammation induced macrophages as 100%, the NO generation inhibition rate was calculated and presented in Table 7.

**[Table 7]**

| Example | Grades of NO generation inhibition rate (%) |
|---|---|
| 1* | D |
| 2* | A |
| 3* | A |
| 4 | B |
| 5* | C |
| 6* | B |
| 7* | D |
| 8* | C |
| 9* | C |
| 14* | C |
| 15* | A |
| 16* | C |
| 17* | C |
| 18* | C |
| 20* | A |
| 22* | A |
| 23* | C |
| 24* | A |
| 25 | A |
| 26* | A |
| 27* | D |
| 28* | B |

| | |
|---|---|
| *Compounds not within the claims | |

(As shown in Table 7, the NO generation inhibition rate (%) according to the compounds of Examples was measured and sorted by the following grades:
A: 35 < A ≤ 100;
B: 25 < B ≤ 35;
C: 15 < C ≤ 25; and
D: 5 < D ≤ 15.)

As shown in Table 7, the 2-phenylbenzofuran derivatives of the present invention display at least 30% of NO generation inhibition rate, indicating that the derivatives are excellent in suppressing NO generation, so that they can be effectively used as a pharmaceutical composition for preventing or treating inflammatory disease.

### 2. Inhibition of interleukin-6 (IL-6) generation

Raw 264.7 cells were distributed by the same manner as described in Experimental Example <2-1>, and IL-6 included in the supernatant treated with each sample at different concentrations was measured by enzyme-linked immunosorbent assay (IL-6 ELISA kit, BD Bioscience, San Diego, CA).

Particularly, 10-fold diluted supernatant was distributed in a 96-well plate on which IL-6 antibody was attached, followed by adhesion for 2 hours. The supernatant remaining after the adhesion was washed with a washing buffer. The detection antibody and HRP were adhered on the washed plate for 1 hour. The remaining antibody was washed. Color development was induced by using TMB substrate. The reaction was terminated with sulfuric acid solution. Then, OD₄₅₀ was measured. IL-6 in the supernatant was quantified by using the standard curve made with the standard IL-6 included in the kit. IL-6 suppression rate was calculated by measuring the same concentration that could inhibit IL-6 generation up to 50% in the LPS treated group and the results are shown in Table 8.

**[Table 8]**

| Example | Grades of IC₅₀ on IL-6 generation (µM) |
|---|---|
| 1* | B |
| 2* | A |
| 3* | E |
| 4 | A |
| 5* | E |
| 6* | B |
| 7* | E |
| 8* | E |
| 9* | E |
| 14* | N.D. |
| 15* | C |
| 16* | D |
| 17* | E |
| 18* | E |
| 20* | C |
| 22* | A |
| 23* | E |
| 24* | B |
| 25 | A |
| 26* | A |
| 27* | C |
| 28* | C |

| | |
|---|---|
| * compounds not within the claims | |

(As shown in Table 8, IC₅₀ (µM) on IL-6 generation according to the compounds of Examples was measured and sorted by the following grades:
A: A ≤ 10;
B: 10 < B ≤ 20;
C: 20 < C ≤ 30;
D: 30 < D ≤ 40; and
E: 40 < E ≤ 50.)

As shown in Table 8, the 2-phenylbenzofuran derivatives of the present invention can inhibit interleukin-6 (IL-6) generation at a low concentration, indicating that the derivatives are excellent in suppressing IL-6 generation, so that they can be effectively used as a pharmaceutical composition for preventing or treating inflammatory disease.

### 3. Inhibition of tumor necrosis factor alpha (TNF-alpha)

Raw 264.7 cells were distributed by the same manner as described in Experimental Example <2-1>, which were treated with the samples selected due to their high NO and IL-6 suppressing effect at different concentrations. TNF-alpha included in the supernatant was measured by enzyme-linked immunosorbent assay (TNF-alpha ELISA kit, R&D Systems, MN, USA).

Particularly, 100-fold diluted supernatant was distributed in a 96-well plate on which TNF-alpha antibody was attached, followed by adhesion for 2 hours. The supernatant remaining after the adhesion was washed with a washing buffer. The detection antibody and HRP were adhered on the washed plate for 1 hour. The remaining antibody was washed. Color development was induced by using TMB substrate. The reaction was terminated with sulfuric acid solution. Then, OD₄₅₀ was measured. TNF-alpha in the supernatant was quantified by using the standard curve made with the standard TNF-alpha included in the kit. TNF-alpha suppression rate was calculated by measuring the same concentration that could inhibit TNF-alpha generation up to 50% in the LPS treated group and the results are shown in Table 9.

**[Table 9]**

| Example | Grades of IC₅₀ on TNF-alpha generation (µM) |
|---|---|
| 1* | C |
| 2* | B |
| 4 | A |
| 6* | E |
| 22* | B |
| 24* | C |
| 25 | A |
| 26* | A |
| 28* | A |

| | |
|---|---|
| *Compounds not within the claims | |

(As shown in Table 9, IC₅₀ (µM) on TNF-alpha generation according to the compounds of Examples was measured and sorted by the following grades:
A: 1 < A ≤ 3;
B: 3 < B ≤ 5;
C: 5 < C ≤ 10;
D: 10 < D ≤ 15; and
E: 15 < E ≤ 17.)

As shown in Table 9, the 2-phenylbenzofuran derivatives of the present invention can inhibit TNF-alpha generation at a low concentration, indicating that the derivatives are excellent in suppressing TNF-alpha generation, so that they can be effectively used as a pharmaceutical composition for preventing or treating inflammatory disease.

### <Manufacturing Example 1> Preparation of pharmaceutical formulations

### <1-1> Preparation of powders

| | |
|---|---|
| The derivative of the invention | 2 g |
| Lactose | 1 g |

Powders were prepared by mixing all the above components, which were filled in airtight packs according to the conventional method for preparing powders.

### <1-2> Preparation of tablets

| | |
|---|---|
| The derivative of the invention | 100 mg |
| Corn starch | 100 mg |
| Lactose | 100 mg |
| Magnesium stearate | 2 mg |

Tablets were prepared by mixing all the above components by the conventional method for preparing tablets.

### <1-3> Preparation of capsules

| | |
|---|---|
| The derivative of the invention | 100 mg |
| Corn starch | 100 mg |
| Lactose | 100 mg |
| Magnesium stearate | 2 mg |

Capsules were prepared by mixing all the above components, which were filled in gelatin capsules according to the conventional method for preparing capsules.

### <1-4> Preparation of injectable solutions

| | |
|---|---|
| The derivative of the invention | 10 µg/Mℓ |
| Weak HCl BP | until pH 3.5 |
| Injectable NaCl BP | up to 1 Mℓ |

The compound of the invention was dissolved in proper volume of injectable NaCl BP. pH of the prepared solution was regulated as 3.5 by using weak HCl BP. The volume was adjusted by using injectable NaCl BP. The solution was well mixed and filled in 5 Mℓ type I transparent glass ampoules. The ampoules were sealed by melting the glass of opening, followed by autoclave at 120°C for at least 15 minutes for sterilization.

### <Manufacturing Example 2> Preparation of health food

The derivative of the invention 500 ng

| Vitamin complex | proper amount |
|---|---|
| Vitamin A acetate | 70 µg |
| Vitamin E | 1.0 mg |
| Vitamin B1 | 0.13 mg |
| Vitamin B2 | 0.15 mg |
| Vitamin B6 | 0.5 mg |
| Vitamin B12 | 0.2 µg |
| Vitamin C | 10 mg |
| Biotin | 10 µg |
| Nicotinic acid amide | 1.7 mg |
| Folic acid | 50 µg |
| Calcium pantothenate | 0.5 mg |

| Minerals | proper amount |
|---|---|
| Ferrous sulfate | 1.75 mg |
| Zinc oxide | 0.82 mg |
| Magnesium carbonate | 25.3 mg |
| Potassium phosphate monobasic | 15 mg |
| Potassium phosphate dibasic | 55 mg |
| Potassium citrate | 90 mg |
| Calcium carbonate | 100 mg |
| Magnesium chloride | 24.8 mg |

Vitamins and minerals were mixed according to the preferable composition rate for health food. However, the composition rate can be adjusted. The constituents were mixed according to the conventional method for preparing health food and then the composition for health food was prepared according to the conventional method.

### <Manufacturing Example 3> Preparation of health beverages

The derivative of the invention 500 ng

| | |
|---|---|
| Citric acid | 1000 mg |
| Oligosaccharide | 100 g |
| Maesil (Prunus mume) Extract | 2 g |
| Taurine | 1 g |
| Purified water | up to 900 Mℓ |

The above constituents were mixed according to the conventional method for preparing health beverages. The mixture was heated at 85°C for 1 hour with stirring and then filtered. The filtrate was loaded in 2 liter sterilized containers, which were sealed and sterilized again, stored in a refrigerator until they would be used for the preparation of a composition for health beverages.

The constituents appropriate for favorite beverages were mixed according to the preferred mixing ratio but the composition ratio can be adjusted according to regional and national preferences, etc.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that the scope of the invention is as set forth in the appended Claims.

## Claims

1. A compound selected from the group consisting of:
3-(7-methoxy-2-p-tolylbenzofuran-5-yl)propane-1-ol; and
4-(5-(3-hydroxypropyl)-7-methoxybenzofuran-2-yl)benzene-1,2-diol
or a pharmaceutically acceptable salt thereof.

2. The compound or pharmaceutically acceptable salt of claim 1 for use in preventing or treating an inflammatory disease.

3. A method for preparing a compound according to claim 1 comprising the following steps, as shown in reaction formula 3:
preparing the compound represented by formula 8 by reacting the compound represented by formula 5 with the compound represented by formula 7 in the presence of a base catalyst (step 1);
preparing the compound represented by formula 9 by inducing hydrogenation of the compound represented by formula 8 obtained in step 1 in the presence of an acid using palladium charcoal as a catalyst (step 2); and
preparing the compound represented by formula 1c by reducing the compound represented by formula 9 obtained in step 2 in the presence of sodium borohydride (step 3): 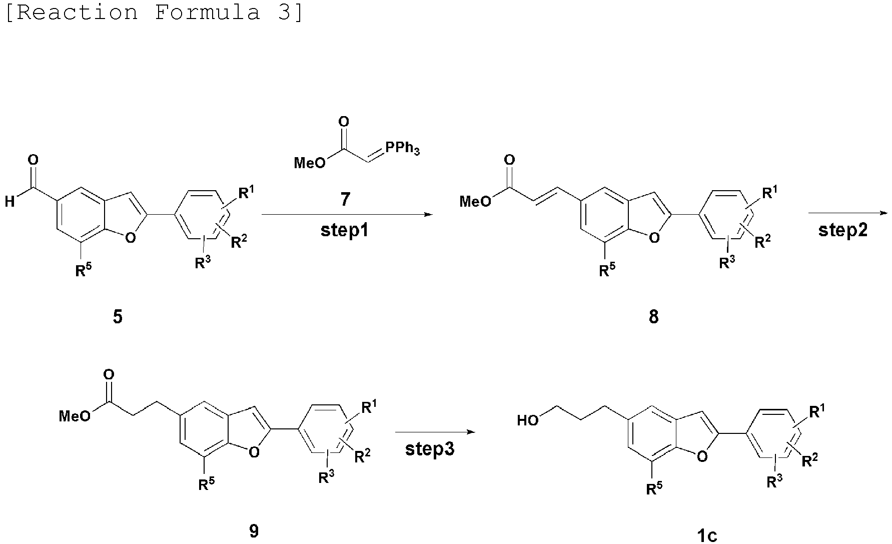 (In the reaction formula 3, R¹, R², R³, and R⁵ are defined by any one of the compounds according to claim 1.)

4. A pharmaceutical composition for use in preventing or treating inflammatory disease comprising the compound according to claim 1 or the pharmaceutically acceptable salt thereof as an active ingredient.

5. The compound or pharmaceutically acceptable salt for use according to claim 2 or the pharmaceutical composition for use according to claim 4, wherein the inflammatory disease is selected from the group consisting of dermatitis, allergy, atopy, conjunctivitis, periodontitis, rhinitis, otitis media, laryngopharyngitis, tonsilitis, pneumonia, gastric ulcer, gastritis, Crohn's disease, colitis, hemorrhoid, gout, ankylosing spondylitis, rheumatic fever, lupus, fibromyalgia, psoriatic arthritis, osteoarthritis, rheumatoid arthritis, periarthritis of shoulder, tendinitis, tenosynovitis, peritendinitis, dermatitis, hepatitis, cystitis, nephritis, Sjogren's syndrome, multiple sclerosis, and acute or chronic inflammatory disease.

6. The pharmaceutical composition for use according to claim 4, wherein the compound according to claim 1 characteristically suppresses the generation of nitric oxide (NO) induced by immune response.

7. The pharmaceutical composition for use according to claim 4, wherein the compound according to claim 1 characteristically suppresses the generation of interleukin-6 (IL-6) induced by immune response.

8. The pharmaceutical composition for use according to claim 4, wherein the compound according to claim 1 characteristically suppresses the generation of TNF-alpha induced by immune response.

9. A health food composition for use in preventing or improving inflammatory disease comprising the compound according to claim 1 or the pharmaceutically acceptable salt thereof as an active ingredient.

## Patentansprüche

1. Eine Verbindung, ausgewählt aus der Gruppe bestehend aus:
3-(7-Methoxy-2-p-tolylbenzofuran-5-yl)propan-1-ol und
4-(5-(3-Hydroxypropyl)-7-methoxybenzofuran-2-yl)benzol-1,2-diol,
oder ein pharmazeutisch annehmbares Salz davon.

2. Die Verbindung oder das pharmazeutisch annehmbare Salz nach Anspruch 1 zur Verwendung zur Verhütung oder Behandlung einer Entzündungserkrankung.

3. Ein Verfahren zur Herstellung einer Verbindung gemäß Anspruch 1, umfassend die folgenden Schritte, wie sie in Reaktionsschema 3 gezeigt sind:
Herstellen der durch Formel 8 dargestellten Verbindung durch Umsetzen der durch Formel 5 dargestellten Verbindung mit der durch Formel 7 dargestellten Verbindung in Gegenwart eines Basenkatalysators (Schritt 1),
Herstellen der durch Formel 9 dargestellten Verbindung durch Herbeiführen einer Hydrierung der durch Formel 8 dargestellten und in Schritt 1 erhaltenen Verbindung in Gegenwart einer Säure unter Verwendung von Palladium-Kohle als Katalysator (Schritt 2), und
Herstellen der durch Formel 1c dargestellten Verbindung durch Reduzieren der durch Formel 9 dargestellten und in Schritt 2 erhaltenen Verbindung in Gegenwart von Natriumborhydrid (Schritt 3): 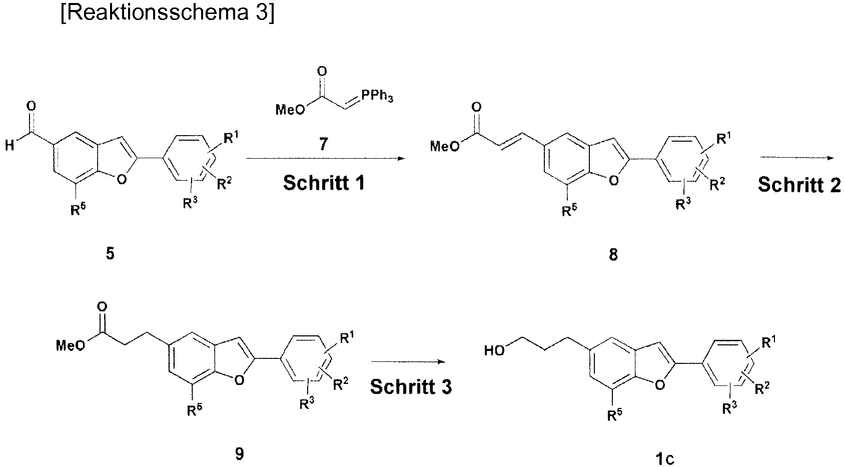 (In dem Reaktionsschema 3 sind R¹, R², R³ und R⁵ durch eine der Verbindungen gemäß Anspruch 1 definiert.)

4. Eine pharmazeutische Zusammensetzung zur Verwendung zur Verhütung oder Behandlung einer Entzündungserkrankung, umfassend die Verbindung gemäß Anspruch 1 oder das pharmazeutisch annehmbare Salz davon als Wirkstoff.

5. Die Verbindung oder das pharmazeutisch annehmbare Salz zur Verwendung gemäß Anspruch 2 oder die pharmazeutische Zusammensetzung zur Verwendung gemäß Anspruch 4, wobei die Entzündungserkrankung ausgewählt ist aus der Gruppe bestehend aus: Dermatitis, Allergie, Atopie, Konjunktivitis, Periodontitis, Rhinitis, Otitis media, Laryngopharyngitis, Tonsillitis, Pneumonie, Magengeschwür, Gastritis, Morbus Crohn, Colitis, Hämorrhoide, Gicht, Spondylitis ankylosans, rheumatisches Fieber, Lupus, Fibromyalgie, Psoriasis-Arthritis, Osteoarthritis, rheumatoide Arthritis, Periarthritis der Schulter, Tendinitis, Tenosynovitis, Peritendinitis, Dermatitis, Hepatitis, Zystitis, Nephritis, Sjögren-Syndrom, Multiple Sklerose und akute oder chronische Entzündungserkrankung.

6. Die pharmazeutische Zusammensetzung zur Verwendung gemäß Anspruch 4, wobei die Verbindung gemäß Anspruch 1 charakteristisch die durch Immunreaktion hervorgerufene Erzeugung von Stickstoffmonoxid (NO) supprimiert.

7. Die pharmazeutische Zusammensetzung zur Verwendung gemäß Anspruch 4, wobei die Verbindung gemäß Anspruch 1 charakteristisch die durch Immunreaktion hervorgerufene Erzeugung von Interleukin-6 (IL-6) supprimiert.

8. Die pharmazeutische Zusammensetzung zur Verwendung gemäß Anspruch 4, wobei die Verbindung gemäß Anspruch 1 charakteristisch die durch Immunreaktion hervorgerufene Erzeugung von TNF-alpha supprimiert.

9. Eine Reformkostzusammensetzung zur Verwendung zur Verhütung oder Besserung einer Entzündungserkrankung, umfassend die Verbindung gemäß Anspruch 1 oder das pharmazeutisch annehmbare Salz davon als Wirkstoff.

## Revendications

1. Composé choisi dans l'ensemble constitué par :
le 3-(7-méthoxy-2-p-tolylbenzofuran-5-yl)propane-1-ol ; et
le 4-(5-(3-hydroxypropyl)-7-méthoxybenzofuran-2-yl)benzène-1,2-diol,
ou un sel de qualité pharmaceutique de celui-ci.

2. Composé ou sel de qualité pharmaceutique selon la revendication 1, pour une utilisation dans la prévention ou le traitement d'une maladie inflammatoire.

3. Procédé pour préparer un composé selon la revendication 1, comprenant les étapes suivantes, comme le montre la formule réactionnelle 3 :
préparation du composé représenté par la formule 8 par réaction du composé représenté par la formule 5 avec le composé représenté par la formule 7 en présence d'un catalyseur basique (étape 1) ;
préparation du composé représenté par la formule 9 par induction d'une hydrogénation du composé représenté par la formule 8 obtenu dans l'étape 1 en présence d'un acide, utilisant du charbon palladié en tant que catalyseur (étape 2) ; et
préparation du composé représenté par la formule 1c par réduction du composé représenté par la formule 9 obtenu dans l'étape 2 en présence de borohydrure de sodium (étape 3) : 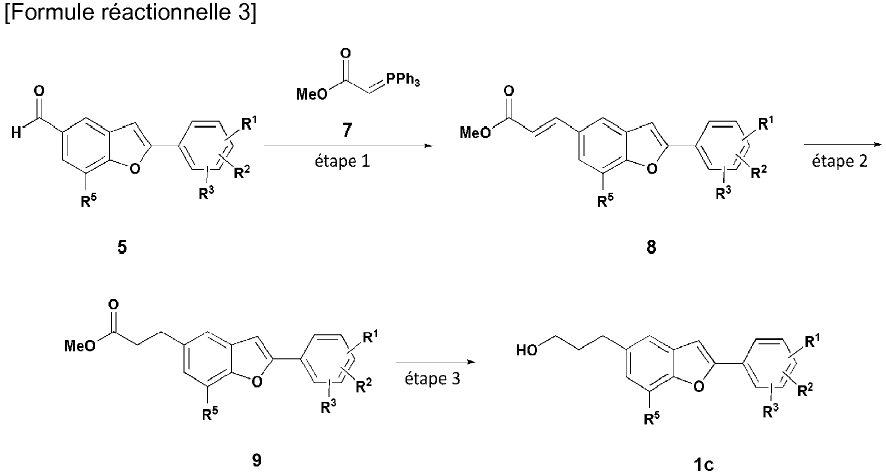 (Dans la formule réactionnelle 3, R¹, R², R³ et R⁵ sont définis par l'un quelconque des composés selon la revendication 1).

4. Composition pharmaceutique pour une utilisation dans la prévention ou le traitement d'une maladie inflammatoire, comprenant, en tant que principe actif, le composé selon la revendication 1 ou son sel de qualité pharmaceutique.

5. Composé ou sel de qualité pharmaceutique pour une utilisation selon la revendication 2 ou composition pharmaceutique pour une utilisation selon la revendication 4, où la maladie inflammatoire est choisie dans l'ensemble constitué par une dermatite, une allergie, une atopie, une conjonctivite, une parodontite, une rhinite, une otite moyenne, une laryngopharyngite, une amygdalite, une pneumonie, un ulcère gastrique, une gastrite, la maladie de Crohn, une colite, des hémorroïdes, la goutte, la spondylarthrite ankylosante, un rhumatisme articulaire aigu, un lupus, la fibromyalgie, l'arthrite psoriasique, l'arthrose, la polyarthrite rhumatoïde, une périarthrite de l'épaule, une tendinite, une ténosynovite, une péritendinite, une dermatite, une hépatite, une cystite, une néphrite, le syndrome de Sjogren, la sclérose en plaques, et une maladie inflammatoire aiguë ou chronique.

6. Composition pharmaceutique pour une utilisation selon la revendication 4, dans laquelle le composé selon la revendication 1 supprime de manière caractéristique la génération d'oxyde nitrique (NO) induite par une réponse immunitaire.

7. Composition pharmaceutique pour une utilisation selon la revendication 4, dans laquelle le composé selon la revendication 1 supprime de manière caractéristique la génération d'interleukine-6 (IL-6) induite par une réponse immunitaire.

8. Composition pharmaceutique pour une utilisation selon la revendication 4, dans laquelle le composé selon la revendication 1 supprime de manière caractéristique la génération de TNF-alpha induite par une réponse immunitaire.

9. Composition d'aliment de santé pour une utilisation dans la prévention ou l'amélioration d'une maladie inflammatoire comprenant, en tant que principe actif, le composé selon la revendication 1 ou son sel de qualité pharmaceutique.
